# EUROPEAN PATENT SPECIFICATION

(11) **EP 1 521 840 B1**
(45) Date of publication and mention of the grant of the patent: **21.04.2010**
(21) Application number: 03764199.0
(22) Date of filing: 14.07.2003
(51) Int. Cl.: C12N 15/87, C12N 15/82, C12M 3/00

(54) **TRANSFORMATION OF PLANT CELLS USING ELECTROPORATION AND DEPRESSURIZATION**
TRANSFORMATION VON PFLANZENZELLEN MITTELS ELEKTROPORATION UND UNTERDRUCK
TRANSFORMATION DE CELLULES VÉGÉTALES PAR ELECTROPORATION ET DEPRESSURISATION

(30) Priority: 16.07.2002 JP 2002207611
(43) Date of publication of application: 13.04.2005
(73) Proprietor: NATIONAL INSTITUTE OF AGROBIOLOGICAL SCIENCES, Tsukuba-shi, Ibaraki 305-8602 (JP)
(72) Inventor: HAGIO, Takashi, c/o NAT. INST. AGROBIO. SCIENCES, Tsukuba-shi, Ibaraki 305-8602 (JP); TABEI, Yutaka, c/o NAT. INST. AGROBIO. SCIENCES, Tsukuba-shi, Ibaraki 305-8602 (JP)
(74) Representative: Wakerley, Helen Rachael
(86) International application number: PCT/JP2003/008937
(87) International publication number: WO 2004/007736

(56) References cited:
- WO-A-00/63407
- WO-A-01/05994
- US-A- 5 422 272
- RHODES C A ET AL: "GENETICALLY TRANSFORMED MAIZE PLANTS FROM PROTOPLASTS" SCIENCE, AMERICAN ASSOCIATION FOR THE ADVANCEMENT OF SCIENCE,, US, vol. 240, 8 April 1988 (1988-04-08), pages 204-207, XP002059218 ISSN: 0036-8075
- SOROKIN ALEXANDER P ET AL: "Production of fertile transgenic wheat plants via tissue electroporation" PLANT SCIENCE (SHANNON), vol. 156, no. 2, July 2000 (2000-07), pages 227-233, XP002261275 ISSN: 0168-9452
- D'HALLUIN KATHLEEN ET AL: "Transgenic maize plants by tissue electroporation" PLANT CELL, vol. 4, no. 12, 1992, pages 1495-1505, XP002015582 ISSN: 1040-4651
- WALDEN R ET AL: "Gene-transfer and plant-regeneration techniques" TRENDS IN BIOTECHNOLOGY, ELSEVIER, AMSTERDAM, NL, vol. 13, no. 9, September 1995 (1995-09), pages 324-331, XP004207196 ISSN: 0167-7799
- RAKOCZY-TROJANOWSKA M.: "Alternative methods of plant transformation - a short review" CELLULAR AND MOLECULAR BIOLOGY LETTERS, vol. 7, 2002, pages 849-858, XP001154761

### SEQUENCE LISTING

<110> National Institute of Agrobiological Sciences
<120> Electroporation method including the use of depressurization/pressurization
<130> AR034PCT
<150> JP P2002-207611
<151> 2002-07-16
<160> 5
<170> PatentIn version 3.1
<210> 1
   <211> 19
   <212> DNA
   <213> Artificial Sequence
<220>
   <223> Description of Artificial Sequence:primer
<400> 1
   ctgcgtgcaa tccatcttg 19
<210> 2
   <211> 23
   <212> DNA
   <213> Artificial Sequence
<220>
   <223> Description of Artificial Sequence:primer
<400> 2
   actcgtcaag aaggcgatag aag 23
<210> 3
   <211> 35
   <212> DNA
   (213) Artificial Sequence
<220>
   <223> Description of Artificial Sequence:primer
<400> 3
   catgattgaa caagatggat tgcacgcagg ttctc 35
<210> 4
   <211> 35
   <212> DNA
   <213> Artificial Sequence
<220>
   <223> Description of Artificial Sequence:primer
<400> 4
   cagaagaact cgtcaagaag gcgatagaag gcgat 35
<210> 5
   <211> 882
   <212> DNA
   (213) Artificial Sequence
<220>
   <223> Description of Artificial Sequence:probe
<400> 5
   tctagaggat cgtttcgcat gattgaacaa gatggattgc acgcaggttc tccggccgct      60
   tgggtggaga ggctattcgg ctatgactgg gcacaacaga caatcggctg ctctgatgcc     120
   gccgtgttcc ggctgtcagc gcaggggcgc ccggttcttt ttgtcaagac cgacctgtcc     180
   ggtgccctga atgaactgca ggacgaggca gcccggctat cgtggctggc cacgacgggc     240
   gttccttgcg cagctgtgct cgacgttgtc actgaagcgg gaagggactg gctgctattg     300
   ggcgaagtgc cggggcagga tctcctgtca tctcaccttg ctcctgccga gaaagtatcc     360
   atcatggctg atgcaatgcg gcggctgcat acgcttgatc cggctacctg cccattcgac     420
   caccaagcga aacatcgcat cgagcgagca cgtactcgga tggaagccgg tcttgtcgat     480
   caggatgatc tggacgaaga gcatcagggg ctcgcgccag ccgaactgtt cgccaggctc     540
   aaggcgcgca tgcccgacgg cgacgatctc gtcgtgaccc atggcgatgc ctgcttgccg     600
   aatatcatgg tggaaaatgg ccgcttttct ggattcatcg actgtggccg gctgggtgtg     660
   gcggaccgct atcatgacat agcgttggct acccgtgata ttgctgaaga gcttggcggc     720
   gaatgggctg accgcttcct cgtgctttac ggtatcgccg ctcccgattc gcagcgcatc     780
   gccttctatc gccttcttga cgagttcttc tgagcgggac tctggggttc gaaatgaccg     840
   accaagcgac gatgaattcg gcggccgcgg ggatcctcta ga                        882

## Description

### TECHNICAL FIELD

The present invention relates to a method for transferring desired nucleic acids into cells or tissue (including plant tissue) using electroporation with depressurization or pressurization to produce nucleic acid-transferred matter (including a transformant).

### BACKGROUND ART

The human race is heavily dependent on major cereals, such as wheat, barley, rice, maize, soybean, and the like, for their survival. To boost production of food to match the worldwide population growth, it is necessary to develop crops having a yield higher than conventional crops. Recombinant DNA techniques have been used to develop transformed crops as a means for breeding varieties having a higher yield.

Vegetables, such as, for example, Chinese cabbage, tomato, cucumber, and the like, are crops which enrich our diet and are vital in terms of nutrition. However, these vegetables are susceptible to various diseases or pathogen damage. If recombinant genetic engineering techniques can be used to confer resistance to disease or pathogen damage, it is possible to achieve stable yields. To meet this demand, methods for isolation of useful genes and transformation using the genes have been developed.

To transform plants, there are generally two methods: the direct gene transfer method, in which a gene is transferred directly into plants; and the indirect gene transfer method, in which a gene is transferred indirectly into plants.

To date, an indirect gene transfer method using *Agrobacterium* has been widely used. For example, rice mature seeds are cultured, and after three weeks, the resultant callus is infected with *Agrobactsrium* (Hiei et al., Plant Journal, 6:271-282, 1994); or seeds at day 4-5 after germination are infected with *Agrobacterium* so that transformants can be rapidlyobtained (Tanaka et al., Japanese Patent No. 3141084).

Examples of direct gene transfer methods include the particle gun method (Christou P. et al., Bio/Technology, 9:957-962, 1991), the polyethylene glycol method (Datta S. K. et al., Bio/Technology, 8:736-740, 1990), an electroporation method (Shimamoto K. et al., Nature, 338:274-276, 1989), and the like. These techniques are utilized for production of transformants. Electroporation is a gene transfer method, in which cells (e.g., plant cells) are placed in a solution containing a desired gene (e.g., DNA), and the gene is introduced into the cells by electrical stimuli.

The direct gene transfer method has an advantage of not requiring tissue culture and preparation of *Agrobacterium,* or the like, over the indirect gene transfer method. However, the particle gun method, which is a direct gene transfer method, has a drawback that the efficiency of regeneration of a transformant (e.g., a transformed whole plant) from transformed tissue is currently low (Hagio, JARQ, 32(4):239-247, 1998). The electroporation method has a drawback that applicable cells and tissue are limited. Therefore, the direct gene transfer method has limited applications.

Conventional electroporation methods are applicable only to cells inherently having no cell wall and cells whose cell wall has been artificially removed (e.g., protoplasts). In contrast, it was believed that electroporation is not possible for cells having a cell wall (e.g., plants (including dormant tissue, such as seeds and the like)). In fact, there has been no report of a method for transferring a nucleic acid to seeds by electroporation. Therefore, in order to use a conventional electroporation method to obtain nucleic acid-transferred matter (particularly, whole transformant plants), protoplast culture, tissue culture (e.g., for redifferentiation) or other steps are unavoidably required after a nucleic acid has been transferred into tissue, such as protoplasts or the like. Therefore, electroporation is not necessarily an easy method, and requires cost, time, and labor.

To transfer a gene into wheat, immature embryos have been used (Weeks J. T. et al. , Plant. Physiol., 102 :1077-1084, 1993). However, the plants need to be grown in fields or green houses in order to obtain immature embryos. In fields, it takes 6 to 7 months. In green houses, it takes 3 to 5 months.

Therefore, there is no available, simple and rapid nucleic acid transfer method (particularly, a transformation method), in which culture and preparation of *Agrobacterium* or the like are not required, and a sample into which a nucleic acid is to be transferred is readily prepared, and nucleic acid-transferred matter (particularly, a transformant) can be readily obtained after nucleic acid transfer.

Considering the above-described situation where no simple and rapid nucleic acid transfer method (particularly, a plant transformation method) has been established, an object of the present invention is to provide a simple and rapid nucleic acid transfer method (particularly, a plant transformation method) having the following features: (i) culture and preparation of *Agrobacterium* or the like are not required; (ii) preparation of a sample into which a nucleic acid is to be transferred is simple; and (iii) nucleic acid-transferred matter can be readily obtained after nucleic acid transfer.

The simple and rapid method of the present invention makes it possible to rapidly obtain a large amount of nucleic acid-transferred matter (particularly, a whole transformant plant). Therefore, the present invention can be utilized in an industrial field requiring plant transformants as well as in research and development using plants to easily allow large-scale processing and large-scale analysis. In addition, the present invention triggers dramatic advances in research, potentially leading to the development of innovative recombinant crops.

### DISCLOSURE OF THE INVENTION

The above-described object of the present invention is achieved by a method for transferring a nucleic acid into a plant cell, comprising the steps of:
a) subjecting the cell to depressurization; and
b) placing the cell and the nucleic acid under conditions capable of inducing electroporation, which includes applying a high voltage pulse to the cell and nucleic acid,
wherein the depressurization step is performed under a pressure reduced by at least 0.02 MPa from atmospheric pressure.

Specifically, various methods for treating cells so that a nucleic acid is easily introduced into the cells have herein been tried by the present inventors to achieve successful electroporation in plants. Conventionally, it is believed that: (i) a nucleic acid transfer method employing *Agrobacterium*, in which plants are placed in vacuum, is not effective for plants other than *Arabidopsis*: and (ii) it is not necessary to hold plants in vacuum so as to transfer a nucleic acid using *Agrobacterium* (Bent, Plant Physiology, 124: 1540-1547, December, 2000). On the contrary, as shown in examples below, the present inventors found that plant nucleic acid transfer by electroporation is optimized by adding the step of holding plant tissue under a pressure different from an atmospheric pressure.

The method of the present invention is considerably simple. In addition, the method of the present invention does not require culture which is conventionally necessary after nucleic acid transfer. Thus, the present invention has an advantage that nucleic acid transformed matter has no somaclonal variation. It is known that a somaclonal variation inevitably occurs in culture which is required after conventional nucleic acid transfer. As is usually understood by those skilled in the art, somaclonal variation refers to a genetic mutation occurring in culture i.e., any sequence alteration (e.g., substitution, deletion, insertion, translocation, inversion, duplication, etc.) occurring in culture in a nucleic acid sequence which is originally possessed by cells into which a nucleic acid is transferred and/or a transferred nucleic acid sequence. An unintended somaclonal variation often confers an undesired trait to nucleic acid-transferred matter. Therefore, since desired nucleic acid-transferred matter can be obtained without a somaclonal variation, the method of the present invention is considerably useful.

The present invention further provides an advantage of permitting transformation of plant species which are impossible or considerably difficult to be transformed by conventional gene transfer techniques. Specifically, Norin 61 (wheat variety), which was used in the examples below, is one of the main varieties of wheat in Japan. It is not possible to regenerate this variety to a whole plant by tissue culture, i.e., it is considerably difficult to obtain a transformant by conventional techniques (Machii et al., Plant Cell, Tissue and Organ Culture, 53:67-74, 1998). Further, for example, a redifferentiation technique has not been well established for soybean which was herein used in Example 4. It was considerably difficult to obtain a transformant of soybean. However, as described in the examples below, the present invention makes it possible to transform plant species which are conventionally impossible or considerably difficult to be made into a transformant. The method of the present invention can be applied to any other species which are conventionally impossible or considerably difficult to be made into a transformant.

Therefore, the present invention provides the following.
1. A method for transferring a nucleic acid into a plant cell, comprising the steps of:
   a) subjecting the cell to depressurization; and
   b) placing the cell and the nucleic acid under conditions capable of inducing electroporation, which includes applying a high voltage pulse to the cell and nucleic acid,
   wherein the depressurization step is performed under a pressure reduced by at least 0.02 MPa from atmospheric pressure.
2. A method according to item 1, in which the plant cell is a cell of dormant plant tissue.
3. A method according to item 2, in which the dormant plant tissue is a seed.
4. A method according to item 1, in which the plant is a monocotyledonous plant.
5. A method according to item 4, in which the monocotyledonous plant is a plant of the family *Gramineae.*
6. A method according to item 5, in which the plant of the family *Gramineae* is wheat (*Triticum aestivum L.*)*.*
7. A method according to item 5, in which the plant of the family *Gramineae* is rice (*Oryza sativa L.*)*.*
8. A method according to item 5, in which the plant of the family *Gramineae* is maize (*Zea mays L.*)*.*
9. A method according to item 1, in which the plant is a dicotyledonous plant.
10. A method according to item 9, in which the dicotyledonous plant is a plant of the family *Cruciferae*.
11. A method according to item 10, in which the plant of the family *Cruciferae* is Chinese cabbage (*Brassica rapa L.*)*.*
12. A method according to item 10, in which the plant of the family *Cruciferae* is rape (*Brassica napus L.*)*.*
13. A method according to item 9, in which the dicotyledonous plant is a plant of the family *Leguminosae.*
14. A method according to item 13, in which the plant of the family *Leguminosae* is soybean (*Glycine max Merr*)*.*
15. A method according to item 9, in which the dicotyledonous plant is a plant of the family *Solanaceae.*
16. A method according to item 15, in which the plant of the family *Solanaceae* is tomato (*Lycopersicum esculentum Mill*).
17. A method according to item 9, in which the dicotyledonous plant is a plant of the family *Cucurbitaceae*.
18. A method according to item 17, in which the plant of the family *Cucurbitaceae* is Japanese cantaloupe (*Cucumis melo L.*)*.*
19. A method according to item 9, in which the dicotyledonous plant is a plant of the family *Convolvulaceae.*
20. A method according to item 19, in which the plant of the family *Convolvulaceae* is morning glory (*Pharbitis nil Choisy*)*.*
21. A method for producing a plant, wherein a nucleic acid is transferred into cells of the plant, comprising the steps of:
   a) subjecting the cell to depressurization; and
   b) placing the cell and the nucleic acid under conditions capable of inducing electroporation, which includes applying a high voltage pulse to the cell and nucleic acid,
      wherein the depressurization step is performed under a pressure reduced by at least 0.02 MPa from atmospheric pressure; and
   c) differentiating, growing and/or multiplying the cell.
22. A method according to item 21, wherein step a) comprises a step of subjecting a seed containing the cell to depressurization, and step b) comprises a step of placing the seed containing the cell and the nucleic acid under the conditions capable of inducing electroporation.
23. A method according to item 22, in which the seed is a monocotyledonous plant seed.
24. A method according to item 23, in which the monocotyledonous plant seed is a seed of the family *Gramineae.*
25. A method according to item 24, in which the seed of the family *Gramineae* is a wheat (*Triticum aestivum L.*) seed.
26. A method according to item 24, in which the seed of the family *Gramineae* is a rice (*Oryza sativa L*.) seed.
27. A method according to item 24, in which the seed of the family *Gramineae* is a maize (*Zea mays L*.) seed.
28. A method according to item 22, in which the seed is a dicotyledonous plant seed.
29. A method according to item 28, in which the dicotyledonous plant seed is a seed of the family *Cruciferae.*
30. A method according to item 29, in which the seed of the family *Cruciferae* is a Chinese cabbage (*Brassica rapa L.*) seed.
31. A method according to item 29, in which the seed of the family *Cruciferae* is a rape (*Brassica napus L*.) seed.
32. A method according to item 28, in which the dicotyledonous plant seed is a seed of the family *Leguminosae*.
33. A method according to item 32, in which the seed of the family *Leguminosae* is a soybean (*Glycine max Merr*) seed.
34. A method according to item 28, in which the dicotyledonous plant seed is a seed of the family *Solanaceae*.
35. A method according to item 34, in which the seed of the family *Solanaceae* is a tomato (*Lycopersicum esculentum Mill*) seed.
36. A method according to item 28, in which the dicotyledonous plant seed is a seed of the family *Cucurbitaceae*.
37. A method according to item 36, in which the seed of the family *Cucurbitaceae* is a Japanese cantaloupe (*Cucumis melo* L.) seed.
38. A method according to item 28, in which the dicotyledonous plant seed is a seed of the family *Convolvulaceae*.
39. A method according to item 38, in which the seed of the family *Convolvulaceae* is a morning glory (*Pharbi tis nil Choisy*) seed.

### BRIEF DESCRIPTION OF THE DRAWINGS

Figure 1 is a restriction map of pWI-H5K.
Figure 2 shows results of wheat seeds (Norin 61) subjected to electroporation under conditions where the pulse width was 50 µsec, the number of pulses was 50, the pressure was 0.096 MPa below an atmospheric pressure (depressurization), and an applied voltage was 100 V (A), 50 V (B), 20 V (C), or 0 V (D).
Figure 3 shows results of GUS staining of 1: a wheat seed subjected to depressurization and electroporation; 2: a wheat seed subjected only to electroporation; and 3: a control wheat seed.
Figure 4 shows results of screening transformed wheat seeds with 2000 ppm geneticin, where A: a sample with a plasmid and B: a control without a plasmid.
Figure 5 shows results of PCR analysis of DNA from transformed wheat plants. Lanes 1 to 8: DNA samples from transformed wheat plants, M: marker, P: positive control, N: negative control. An arrow to the right indicates the position of a product of interest (about 0.75 kb).
Figure 6 shows results of Southern blotting analysis of DNA from transformed wheat plants. Lanes 1 to 8: DNA samples from transformed wheat plants, P1: positive control, P2: positive control, N: negative control. Arrows to the right from the top indicate about 4.0 kb, about 2.0 kb, about 1.5 kb, and about 1.0 kb.
Figure 7 shows a photograph of wheat transformants having seed fertility. The plants in the photograph correspond to the plants of lanes 1 and 2 which were positive in Southern blotting analysis of Figure 6.
Figure 8 shows results of PCR analysis of DNA from the next generation individuals (T1) of transformed wheat plants. Lanes 1 to 6: DNA samples from the next generation individuals (T1) of transformed wheat plants, M: marker, P: positive control, N: negative control. An arrow to the right indicates the position of a product of interest (about 0.75 kb).
Figure 9 shows results of rice seeds (Japonica) subjected to electroporation under conditions where the pulse width was 50 µsec, the number of pulses was 99, the pressure was 0.096 MPa below an atmospheric pressure (depressurization), and an applied voltage was 50 V (A), 20 V (B), 10 V (C), or 0 V (D).
Figure 10 shows results of GUS staining of 1: a Japonica rice seed subjected to depressurization and electroporation; 2: a Japonica rice seed subjected only to electroporation; and 3: a control Japonica rice seed.
Figure 11 shows results of screening transformed Japonica rice seeds with 200 ppm geneticin, where A: a sample with a plasmid and B: a control without a plasmid.
Figure 12 shows results of Southern blotting analysis of DNA from transformed Japonica rice plants . Lanes 1 to 6: DNA samples from transformed Japonica rice plants, P: positive control (corresponding to about 5 copies), N: negative control. An arrow to the right indicates the position of a product of interest (about 0.8 kb).
Figure 13 shows a photograph of Japonica rice transformants having seed fertility. The plants in the photograph correspond to the plants of lane 5 which were positive in Southern blotting analysis of Figure 12.
Figure 14 shows results of PCR analysis of DNA from the next generation individuals (T1) of transformed Japonica rice plants. Lanes 1 to 8: DNA samples from the next generation individuals (T1) of transformed Japonica rice plants, M: marker, P: positive control, N: negative control. An arrow to the right indicates the position of a product of interest (about 0.75 kb).
Figure 15 shows results of GUS staining of A: an Indica rice seed subjected to depressurization and electroporation; B: an Indica rice seed subjected only to electroporation; and C: a control Indica rice seed.
Figure 16 shows results of GUS staining of 1: a soybean seed subjected to depressurization and electroporation? and 2: a control soybean seed.
Figure 17 shows results of GUS staining of A: Chinese cabbage seeds subjected to depressurization and electroporation; and B: control Chinese cabbage seeds.
Figure 18 shows results of GUS staining of A: an tomato seed subjected to depressurization and electroporation; B: a tomato seed subjected only to electroporation; and C: a control tomato seed.
Figure 19 shows results of GUS staining of A: a morning glory seed subjected to depressurization and electroporation; and B: a control morning glory seed.
Figure 20 shows results of wheat seeds subjected to electroporation where the voltage was 100 V, the pulse width was 50 µsec, the number of pulses was 50, and A: a pressure 0.096 MPa below an atmospheric pressure, B: a pressure 0.06 MPa below the atmospheric pressure, C: no depressurization, or D: a control (without DNA and depressurization).
Figure 21 shows results of Japonica rice seeds subjected to electroporation where the voltage was 50 V, the pulse width was 50 µsec, the number of pulses was 99, and A: a pressure 0.096 MPa below an atmospheric pressure, B: a pressure 0.06 MPa below the atmospheric pressure, C: no depressurization, or D: a control (without DNA and depressurization).
Figure 22 shows an example of electroporation apparatus suitable for carrying out the method of the present invention.
Figure 23 shows another example of electroporation apparatus suitable for carrying out the method of the present invention.
Figure 24 shows another example of electroporation apparatus suitable for carrying out the method of the present invention.
Figure 25 shows an example of a rectangular parallelepiped shaped electroporation chamber suitable for use in the method of the present invention.
Figure 26 shows an example of a microtube shaped electroporation chamber suitable for use in the method of the present invention.
Figure 27 shows an example of the largest inscribed circle touching at least three points on an inner wall of the exemplified electroporation chamber used in the present invention.
Figure 28 shows a diagrammatic illustration of automatic electroporation apparatus suitable for carrying out the method of the present invention.
Figure 29 shows an example of automatic electroporation apparatus suitable for carrying out the method of the present invention.

### BEST MODE FOR CARRYING OUT THE INVENTION

Hereinafter, the present invention will be described by way of illustrative examples with reference to the accompanying drawings.

It should be understood throughout the present specification that articles for singular forms include the concept of their plurality unless otherwise mentioned. It should be also understood that terms as used herein have definitions ordinarily used in the art unless otherwise mentioned.

The following terms as used in this disclosure have the meanings ascribed to them below.

As used herein, the term "nucleic acid transfer" refers to transferring a nucleic acid into cells or tissue in an artificial manner. The phenotype of cells or tissue, into which a nucleic acid has been transferred by "nucleic acid transfer", may or may not alter. As used herein, the term "gene transfer" refers to transferring a nucleic acid containing a gene, which is a factor for determining a hereditary trait, into cells or tissue in an artificial manner. The phenotype of cells or tissue, into which a nucleic acid containing a gene has been transferred by "gene transfer", may or may not alter. As used herein, the term "transformation" refers to alteration of the phenotype of cells or tissue by introduction of a nucleic acid containing a gene into the cells or tissue. Note that, in the particular situation, the terms "nucleic acid transfer" , "gene transfer", and "transformation" may be herein used interchangeably. The meanings of these respective terms are clearly understood by those skilled in the art in the context of the present specification.

The terms "nucleic acid-transferred matter", "gene-transferred matter", and "transformant" refer to the whole or part of an organism generated from a cell or tissue undergoing nucleic acid transfer, gene transfer, and transformation, respectively. Note that, in the particular situation, the terms "nucleic acid-transferred matter", "gene-transferred matter", and "transformant" may be herein used interchangeably. The meanings of these respective terms are clearly understood by those skilled in the art in the context of the present specification. Nucleic acid-transferred matter, gene-transferred matter, and transformants may be any organisms, including, for example, organisms generated from prokaryotic cells and eukaryotic cells (e.g., plant cells, etc.) or tissue. A transformant is also referred to as a transformed cell, a transformed tissue, a transformed host, or the like, depending on what is transformed. As used herein, the term "transformant" encompasses all of these forms and may refer to a particular form in the particular context. The same applies to the terms "nucleic acid-transferred matter" and "gene-transferred matter".

The term "cell" refers to a cell from any organism (e.g., any type of multicellular organism (e.g., an animal (e.g., a vertebrate, an invertebrate), a plant (e.g., a monocotyledonous plant, a dicotyledonous plant, etc.), a fungus, etc.), a unicellular organism (e.g., a bacteria, etc.)). Preferably, cells used in the present invention are ones which have a cell wall, particularly preferably plant cells.

As used herein, "tissue" refers to an aggregation of cells having substantially the same function and/or form in a multicellular organism. Tissue is typically an aggregation of cells derived from the same origin, or may be an aggregation of cells derived from different origins if the cells have substantially the same function and/or form. Typically, tissue is a part of an organ. In plants, tissue is roughly divided in various manners, for example, into meristematic tissue and permanent tissue, depending on the developmental state of the element cells; or simple tissue and complex tissue, depending on the type of element cells. Animal tissue is divided into epithelial tissue, connective tissue, muscular tissue, nervous tissue, and the like, depending on the form, function or genetic basis.

As used herein, the term "issue" refers to any tissue derived from any organism (e.g., any type of multicellular organism (e.g., an animal (e.g., a vertebrate, an invertebrate), a plant (e.g., a monocotyledonous plant, a dicotyledonous plant, etc.), a fungus, etc.). Preferably, tissue used in the present invention is one which has a cell wall, particularly preferably plant tissue. Examples of plant tissue include, but are not limited to, dormant tissue, germ plasma, a growing point, and a flower bud. Preferable examples of dormant tissue include, but are not limited to, a mature seed, an immature seed, a winter bud, and a tuber. Particularly preferable dormant tissue is a mature seed, but is not limited to this.

As used herein, the term "organ" refers to a structure which is a specific portion of an individual organism where a certain function of the individual organism is locally performed and which is morphologically independent. Generally, in multicellular organisms (e.g., animals, plants, fungi), organs are made of several tissues in specific spatial arrangement and tissue is made of a number of cells. Examples of plant organs include, but are not limited to, root, leaf, stem, flower, and the like. Examples of animal organs include, but are not limited to, skin, heart, blood vessel, cornea, retina, kidney, liver, pancreas, intestine, placenta, umbilical cord, lung, brain, nerve, peripheral limb, and the like.

As used herein, the term "screening" refers to distinguishing nucleic acid-transferred matter from non-nucleic acid-transferred matter by an antibiotic-resistance test and/or genetic engineering techniques (e.g., PCR, Southern blotting, northern blotting, and the like). In particular, the term "screening" refers to a step of distinguishing transformed organisms having an introduced drug-resistance gene from untransformed organisms by culturing and/or growing these organisms in the presence of a drug to which the transformed organisms have resistance.

As used herein, the term "electroporation" refers to a technique for transferring nucleic acid (e.g., a nucleic acid containing a gene) into cells (e.g., plant cells), in which a DC high voltage pulse is applied to the cells to open pores allowing the nucleic acid to enter the cells. Conditions for electroporation may be appropriately selected by those skilled in the art, depending on the species, tissue, cells, or the like, which are used. A typical voltage used for electroporation is 10 V/cm to 200 V/cm, preferably 20 V/cm to 150 V/cm, more preferably 30 V/cm to 120 V/cm, even more preferably 40 V/cm to 100 V/cm, and most preferably 50 V/cm to 100 V/cm, but is not limited to these values. A typical pulse width for electroporation is 1 µsec to 90 µsec, preferably 10 µsec to 90 µsec, still preferably 20 µsec to 80 µsec, still more preferably 30 µsec to 80 µsec, still even more preferably 40 µsec to 70 µsec, and most preferably 50 µsec to 60 µsec, but is not limited to these values. A typical number of pulses for electroporation is 1 to 200, preferably 10 to 150, more preferably 20 to 120, even more preferably 30 to 110, and most preferably 40 to 100, but is not limited to these values.

As used herein, the phrase "placing a cell (or tissue) and a nucleic acid under conditions capable of inducing electroporation" refers to placing a cell (or tissue) and a nucleic acid in a state which has all conditions (a voltage, a pulse width, the number of pulses, the positional relationship between a cell (or tissue) and a nucleic acid, the running time of electroporation, and the like) essential for inducing electroporation (i.e., the transfer of a nucleic acid into a cell (or tissue)). Conditions essential for electroporation are clearly known to those skilled in the art and may be appropriately determined by those skilled in the art.

In the present invention, electroporation is preferably performed by applying a high voltage pulse to a cell (or tissue) and a nucleic acid in at least two directions. This technique may be achieved most simply by applying a high voltage pulse to a cell (or tissue) and a nucleic acid for a predetermined period of time, and thereafter, exchanging the positions of the anode electrode and the cathode electrode and applying a high voltage pulse again. This technique may also be achieved using electrode pairs disposed at different positions in an electroporation chamber. By applying a high voltage pulse in at least two directions, the efficiency of transferring of a nucleic acid can be significantly improved. For example, the present inventors found that when a high voltage pulse is applied to a *Cruciferae* plant seed and a nucleic acid in two directions, the efficiency of transferring of the nucleic acid into the seed was improved by a factor of about two or more than when a high voltage pulse was applied in only one direction.

An electroporation chamber used when electroporation is performed in the present invention may have any dimensions as long as the chamber can accommodate a cell and/or tissue which is subjected to nucleic acid transfer. Particularly preferably, an electroporation chamber has a size which allows plant tissue (e. g., a plant seed) to be accommodated. The electroporation chamber may be of any shape. Examples of such a shape include, but are not limited to, a cube, a rectangular parallelepiped, a cylinder, a tube (e. g., the body has a uniform or non-uniform horizontal section, and the body may or may not taper toward the bottom), and the like. In order for an electroporation chamber to accommodate a plant seed, the diameter of a largest inscribed circle touching at least three points on the inner wall of the chamber may be, for example, at least about 5 mm or more, preferably at least about 6 mm or more, preferably at least about 7 mm or more, preferably at least about 8 mm or more, preferably at least about 9 mm or more, preferably at least about 1 cm or more, preferably at least about 2 cm or more, preferably at least about 3 cm or more, preferably at least about 4 cm or more, preferably at least about 5 cm or more, preferably at least about 6 cm or more, preferably at least about 7 cm or more, preferably at least about 8 cm or more, preferably at least about 9 cm or more, preferably at least about 10 cm or more, preferably at least about 15 cm or more, and preferably at least about 20 cm or more. The upper limit of the diameter of a largest inscribed circle touching at least three points on the inner wall of an electroporation chamber may be, but is not limited to, for example, about 25 cm, about 20 cm, about 15 cm, about 10 cm, about 9 cm, about 8 cm, about 7 cm, about 6 cm, about 5 cm, about 4 cm, about 3 cm, about 2 cm, about 1 cm, about 9 mm, about 8 mm, about 7 mm, or about 6 mm. The length may be an intermediate value between these explicitly described values (e. g., 1.5 cm, etc.). As used herein, the term "inscribed circle" refers to any circle touching at least three arbitrary points on the inner wall of a chamber container. Here, the electrodes provided in the chamber are also regarded as a portion of the container. Therefore, the inner wall of the chamber container includes a surface of the electrode. Typically, the thickness of the electrode is negligibly small (e. g., 0.1 mm or the like).

One example of an electroporation chamber suitable for carrying out the method of the present invention has a quadrangular horizontal section and inner dimensions, 1 cm x 2 cm x 2 cm (e. g., length x width x height). In another example, the electroporation chamber has a circular horizontal section and inner dimensions, 1 cm x 4 cm (e. g., diameter x height). The regions occupied by the electrodes are not included in the dimensions of the chamber. Typically, the thickness of the electrode is negligibly small. As used herein, the term "horizontal section" refers to a cross section perpendicular to the longitudinal direction of a chamber. As used herein, the term "inner dimension" refers to a distance between two arbitrary points on the inner wall of a chamber container. When a chamber has a quadrangular horizontal section, an inner dimension refers to the length or width of the horizontal section, or height. When a chamber has a circular horizontal section, an inner dimension refers to the diameter of the horizontal section, or height.

The electroporation chamber may be changed to a size which allows the chamber to accommodate a plant seed. The size of the chamber may be changed by any means. For example, the size of the chamber may be adjusted to appropriate dimension (s) by means of a screw or the like.

The electroporation chamber may be formed of any material. A material for the electroporation chamber may be any material which can form a solid. Examples of such a material include, but are not limited to, glass, silica, silicon, ceramics, silicon dioxide, plastics, metals (including alloys), native and synthetic polymers (e.g., polystyrene, cellulose, chitosan, dextran, and nylon), and the like. The chamber may be formed of layers made of different materials. For example, inorganic insulating materials, such as glass, quartz glass, alumina, sapphire, forsterite, silicon oxide, silicon carbide, silicon nitride, and the like, can be used. Organic materials, such as polyamide, polycarbonate, (denatured) polyphenylene oxide, polybutylene terephthalate, reinforced polyethylene terephthalate, polyether sulfone, polyphenylene sulfide, polyarylate, polyether imide, polyether etherketone, polyimide, polyester, polyethylene, polypropylene, polyisobutylene, unsaturated polyester, fluororesin, polyvinyl chloride, polyvinylidene chloride, polyvinyl acetate, polyvinyl alcohol, polyvinyl acetal, acrylic resin, polyacrylonitrile, polystyrene, acetal resin, phenol resin, urea resin, epoxy resin, melamine resin, styrene-acrylonitrile copolymer, acrylonitrile-butadiene -styrene copolymer, silicone resin, polysulfone, and the like, may be used. In addition, preferably, the electroporation chamber is capable of resisting a pressure different from an atmospheric pressure (e. g., even when a pressure different from an atmospheric pressure is applied to the chamber, the chamber would not break, rupture or deform). Particularly preferably, the chamber is capable of resisting depressurization. Particularly preferably, the electroporation chamber is formed of polypropylene, silicone resin, and glass, and is provided with electrodes made of platinum or stainless steel.

Preferably, the electroporation chamber used to carry out the method of the present invention is provided with a temperature control section. For example, the temperature control section may sense a change in temperature using a sensor and may manually or automatically control the temperature of the chamber. The temperature control section is representatively a cooling section for reducing the temperature of the chamber. The cooling section may be any means which utilizes, for example, ice, cooling gel, or the like.

The electroporation chamber used to carry out the method of the present invention is provided with at least a pair of (two) electrodes. Therefore, the chamber may be provided with more than a pair of (two) electrodes (e. g., two pairs of (four) electrodes, three pairs of (six) electrodes, four pairs of (eight) electrodes, five pairs of (ten) electrodes, or more than five pairs of electrodes). For example, in the case of a chamber having a quadrangular horizontal cross section, if an electrode is provided along each of the facing inner side walls, two pairs of (four) electrodes can be attached to the chamber. Further, in the case of a chamber having a hexagonal cross section, if an electrode is provided along each of the facing inner side walls, three pairs of (six) electrodes can be attached to the chamber. Thus, an arbitrary number of pairs of electrodes may be provided in the chamber. Also, the electrodes may have an arbitrary spatial positional relationship.

The distance between electrodes provided in the electroporation chamber used to carry out the method of the present invention may have any length and may vary depending on the size of a cell and/or tissue which is subject to nucleic acid transfer. Particularly preferably, the distance between electrodes is enough to accommodate plant tissue (e. g., a plant seed). The enough distance between electrodes to accommodate a plant seed may be, for example, at least about 5mm or more, preferably at least about 6 mm or more, preferably at least about 7 mm or more, preferably at least about 8 mm or more, preferably at least about 9 mm or more, preferably at least about 1 cm or more, preferably at least about 2 cm or more, preferably at least about 3 cm or more, preferably at least about 4 cm or more, preferably at least about 5 cm or more, preferably at least about 6 cm or more, preferably at least about 7 cm or more, preferably at least about 8 cm or more, preferably at least about 9 cm or more, preferably at least about 10 cm or more, preferably at least about 15 cm or more, and preferably at least about 20 cm or more. The upper limit of the distance between electrodes may be, but is not limited to, for example, about 25 cm, about 20 cm, about 15 cm, about 10 cm, about 9 cm, about 8 cm, about 7 cm, about 6 cm, about 5 cm, about 4 cm, about 3 cm, about 2 cm, about 1 cm, about 9 mm, about 8 mm, about 7 mm, or about 6 mm. The length may be an intermediate value between these explicitly described values (e. g., 1.5 cm, etc.).

The distance between electrodes may be changed so that a plant seed can be accommodated between the electrodes. The distance between electrodes may be changed by any means. For example, the distance between electrodes may be adjusted to an appropriate distance by means of a screw or the like.

The electrode may be made of any material as long as the material has ability to conduct electricity. Examples of a material for the electrode include, but are not limited to, for example, platinum, gold, stainless steel, carbon, and conductive polymers. Particularly preferably, the electrode is made of platinum.

An illustrative electroporation chamber is in the shape of a rectangular parallelepiped of length 1 cm x width 2 cm x height 2 cm and is provided with platinum electrodes, where the distance between the platinum electrodes is about 1 cm. This electroporation chamber is particular useful for a plant seed (e. g., wheat, rice, maize, etc.) having an intermediate size (about 5 to 15 mm). With this chamber, a number of (e. g., about 10 to 30 grains) intermediate-size plant seeds can be simultaneously treated.

Another illustrative electroporation chamber is in the shape of a microtube of inner diameter 1 cm x height 4 cm and is provided with stainless electrodes, where the distance between the stainless electrodes is about 1 cm. This microtube type chamber may be readily produced by attaching a stainless foil (e.g., about 5 x 40 mm (thickness: about 0.1 mm)) to the inner wall of a commercially available microtube with an adhesive or the like. The microtube type chamber can be subjected to centrifugation so that cell, tissue and/or seeds can be precipitated at the bottom of the chamber. Therefore, the exchange of solutions can be readily performed. The microtube type chamber is useful particularly when a small size (about 0.1 to 5 mm) plant seed (e.g., *Arabidopsis thaliana,* etc.) is treated. With this chamber, a number of small-size plant seeds can be simultaneously treated.

As used herein, "holding a cell/tissue (including plant tissue) under a pressure different from an atmospheric pressure" refers to a step of holding a cell/tissue (including plant tissue) under a pressure higher (pressurization) or lower (depressurization) than an atmospheric pressure (typically, 1 atmospheric pressure = 101.325 kPa = about 0.1 MPa).

Though not wishing to be limited by any particular theory, it is considered that by holding a cell/tissue (including plant tissue) under a pressure different from an atmospheric pressure, the ambient pressure applied to the cell/tissue is changed so that a buffer solution containing a nucleic acid, such as DNA or the like, can permeate tissue or cavities between cells; and as a result, it becomes possible to undergo nucleic acid transfer/transformation by electroporation into cells and tissue having a cell wall (particularly, plant cells and plant tissue), which is conventionally considered to be impossible.

As used herein, the term "depressurization" refers to a step of holding a cell/tissue (including plant tissue (e. g., a seed)) under a pressure lower than an atmospheric pressure for nucleic acid transfer/transformation. In the present invention, depressurization is performed under a pressure lower by at least 0.02 MPa than an atmospheric pressure, preferably a pressure lower by 0.04 MPa, more preferably a pressure lower by 0.06 MPa, still more preferably a pressure lower by 0.08 MPa, and most preferably a pressure lower by 0.096 MPa than an atmospheric pressure. The running time of depressurization is, but is not limited to, 1 min to 120 min, preferably 10 min to 100 min, more preferably 15 min to 90 min, still more preferably 30 min to 70 min, and most preferably about 60 min.

As used herein, the term "pressurization" refers to a step of holding a cell/tissue (including plant tissue (e. g., a seed)) under a pressure higher than an atmospheric pressure for nucleic acid transfer/transformation.

The electroporation apparatus used to carry out the method of the present invention can transfer a nucleic acid into any cell or tissue with significantly high efficiency, and particularly, is useful for nucleic acid transfer into a cell or tissue having a cell wall (e. g., a plant cell or plant tissue) which is conventionally impossible for nucleic acid transfer by electroporation. The electroporation apparatus may comprise both a) a section for holding a cell under a pressure different from an atmospheric pressure and b) an electroporation section.

The section for holding a cell under a pressure different from an atmospheric pressure may be any means capable of performing depressurization and/or pressurization. A commercially available depressurization apparatus (e. g., a vacuum desiccator, etc.) and/or a pressurization apparatus may also be utilized. Any electroporation means may be utilized. A commercially available electroporation means (e. g., CUY21EDIT gene transfer apparatus, Nepagene, Ichikawa-shi, Chiba-ken, Japan) may be utilized. Preferably, the distance between two electrodes (first electrode and second electrode) provided in the electroporation section is enough to accommodate a plant seed, as defined above.

An electroporation apparatus comprising two electrodes may be used, where the distance therebetween is enough to accommodate a plant seed. The electroporation apparatus is used in combination with means for holding a cell/tissue under a pressure different from an atmospheric pressure. The electroporation apparatus and the section for holding a cell/tissue under a pressure different from an atmospheric pressure are not necessarily present in the same housing.

Conventional electroporation apparatuses aim to apply a high voltage pulse to a considerably small cell. Therefore, the inner dimensions of the chamber and the distance between electrodes need to be minimized to as small as possible. Therefore, the inner dimensions of the chamber and the distance between electrodes in conventional electroporation apparatuses are typically about 1 mm or 2 mm, or 4 mm at most. Therefore, an electroporation apparatus comprising a chamber having enough space to accommodate a plant seed and electrodes having an enough distance therebetween to accommodate a plant seed as set forth herein has never been heretofore known.

Further, the electroporation apparatus used to carry out the present invention may be automatically operated. In an example of automatic electroporation apparatus suitable for carrying out the method of the present invention, the injection and/or exchange of buffer solutions or the like may be performed by an automatic dispensing machine. As an automatic dispensing machine, for example, a commercially available epMotion5070 workstation (Eppendorf Co., Ltd., Higashi-Kanda 3, Chiyoda-ku, Tokyo, Japan) or the like may be used. An automatic dispensing machine is not limited to this. Particularly, an automatic dispensing machine may be advantageously used as a section for placing a nucleic acid and/or a cell in a container for placing a mixture containing a nucleic acid and a cell.

A first container for placing a mixture containing a nucleic acid and a cell, a second container for holding the cell under a pressure different from an atmospheric pressure, and a third container for applying a high voltage pulse to the mixture containing the nucleic acid and a cell, may be any containers. These containers may be the same or different from one another. A material for these containers may be any material capable of forming a solid. Examples of such a material include, but are not limited to, glass, silica, silicon, ceramics, silicon dioxide, plastics, metals (including alloys), native and synthetic polymers (e. g., polystyrene, cellulose, chitosan, dextran, and nylon), and the like. The container may be formed of layers made of different materials. For example, inorganic insulating materials, such as glass, quartz glass, alumina, sapphire, forsterite, silicon oxide, silicon carbide, silicon nitride, and the like, can be used. Organic materials, such as polyamide, polycarbonate, (denatured) polyphenylene oxide, polybutylene terephthalate, reinforced polyethylene terephthalate, polyether sulfone, polyphenylene sulfide, polyarylate, polyether imide, polyether etherketone, polyimide, polyester, polyethylene, polypropylene, polyisobutylene, unsaturated polyester, fluororesin, polyvinyl chloride, polyvinylidene chloride, polyvinyl acetate, polyvinyl alcohol, polyvinyl acetal, acrylic resin, polyacrylonitrile, polystyrene, acetal resin, phenol resin, urea resin, epoxy resin, melamine resin, styrene-acrylonitrile copolymer, acrylonitrile-butadienestyrene copolymer, silicone resin, polysulfone, and the like, may be used.

Preferably, a first container is produced from a material (e.g., polystyrene) which is highly transparent and facilitates observation of samples. Preferably, a second container is produced from a material (e. g., polyamide, polycarbonate, (denatured) polyphenylene oxide, polybutylene terephthalate, reinforced polyethylene terephthalate, polyether sulfone, polyphenylene sulfide, polyarylate, polyether imide, polyether etherketone, polyimide, and epoxy resin) which is capable of resisting a pressure different from an atmospheric pressure (particularly, depressurization), and more preferably a material (e. g., acrylic resin) which is highly transparent and facilitates observation of samples. Preferably, a third container is produced from a material (e. g., polypropylene, silicone resin, and glass) which has affinity for cells, and is provided with electrodes made of platinum, gold, stainless steel, carbon, or a conductive polymer. These materials may be coated with any appropriate material in order to provide a desired property (e. g., insulation of a container, improved conductivity of electrodes, etc.).

In addition, preferably, the first and second containers for use in the method of the present invention are capable of resisting a pressure different from an atmospheric pressure. Particularly preferably, the first and second containers are capable of resisting depressurization. For example, the first container may be housed in the second and/or third container. Alternatively, a mixture containing a nucleic acid and a cell is injected from the first container to the second container, (or another container housed therein) and/or the third container (or another container housed therein).

As a section for placing a cell in the second container and a section for placing a mixture containing a nucleic acid and a cell in the third container, a belt conveyor or the like may be advantageously used. Any means may be used. Liquids placed in the first container, the second container, and the third container may be moved by suction/drainage utilizing an automatic pump or the like.

Automatic electroporation apparatus suitable for carrying out the method of the present invention comprises a control apparatus for automating various operational means. The electroporation apparatus comprises a power supply apparatus. The control apparatus and the power supply apparatus may be installed in the same housing which contains an electroporation apparatus, or may be separated from an electroporation apparatus and may be connected to the electroporation apparatus.

When a seed is subjected to electroporation, preferably, the seed is immersed in water (e. g., tap water) before depressurization or pressurization. The period of time during which the seed is immersed in water before the treatment is, but is not limited to, 6 h to 48 h, preferably 12 h to 36 h, more preferably 18 h to 30 h, even more preferably 20 h to 26 h, and the most preferably about 24 h.

Illustrative conditions for the nucleic acid transfer of the present invention are described as follows. A seed is immersed in tap water at 25°C over night. On the next day, the seed is placed in a vacuum apparatus, followed by depressurization under a pressure lower by 0.096 MPa, than an atmospheric pressure. Thereafter, a high voltage pulse (100 V, 50 µsec, the distance between electrodes: 1 cm) is applied about 50 times to the seed treated by depressurization for electroporation, i.e., nucleic acid transfer/gene transfer. The voltage and the number of pulses may vary depending on the crop. Conditions for electroporation may be appropriately selected by those skilled in the art if required. Thereafter, cells or tissue are screened in a medium containing antibiotics, and thereafter, are potted (raised in a pot), thereby making it possible to obtain a normal whole plant.

As used herein, the term "plant" is a generic term encompassing organisms belonging to the plant kingdom, characteristically containing chloroplasts, having rigid cell walls, permanently producing abundant embryonic tissue, and lacking the power of locomotion. Plants are categorized in, for example, in "Genshoku Makino Shokubutsu Daizukan [Unabridged Makino's Original Color Illustrated Reference Book of Plants], Hokuryukan (1982), Japan), and the like. All plants described therein may be used in the present invention. Representatively, a plant refers to a flowering plant which forms cell walls and has anabolism by chloroplast. "Plant" includes any of monocotyledonous and dicotyledonous plants. Examples of monocotyledonous plants include plants of the family *Gramineae.* Examples of preferable monocotyledonous plants include, but are not limited to, maize, wheat, rice, oat, barley, sorghum, rye, and millet, andmorepreferablymaize, wheat, and rice. Examples of wheat include wheat variety Norin 61, which was difficult to transform by conventional methods. Examples of dicotyledonous plants include, but are not limited to, plants of the family *Cruciferae, Leguminosae, Solanaceae, Cucurbitaceae,* and *Convolvulaceae.* Examples of plants of the family *Cruciferae* include, but are not limited to, Chinese cabbage, rape, cabbage, and cauliflower. Preferable *Cruciferae* plants are Chinese cabbage and rape. A particularly preferable *Cruciferae* plant is rape. Examples of plants of the family *Leguminosae* include, but are not limited to, soybean, azuki bean, kidney bean, and cowpea. A preferable *Leguminosae* plant is soybean. Examples of plants of the family *Solanaceas* include, but are not limited to, tomato, egg plant, and potato. A preferable *Solanaceae* plant is tomato. Examples of plants of the family *Cucurbitaceae* include, but are not limited to, Japanese cantaloupe, cucumber, melon, and water melon. A preferable *Cucurbitaceae* plant is Japanese cantaloupe. Examples of plants of the family *Convolvulaceae* include, but are not limited to, morning glory, sweet potato, and bellbind. A preferable *Convolvulaceae* plant is morning glory. A plant means any of whole plants, plant organs, plant tissues, plant cells and seeds unless otherwise specified. Examples of plant organs include root, leaf, stem, flower, and the like. Examples of plant cells include callus and a suspension of cultured cells.

In one embodiment of the present invention, plants of the families *Solanaceae, Gramineae*, *Cruciferae, Rosaceae*, *Leguminosae, Cucurbitaceae, Lamiacea*, *Liliaceae*, *Chenopodiaceae, Umbelliferae, Convolvulaceae, Compositae,* and the like, are used, for example. Examples of plant species used in the present invention include any tree species, any fruit tree species, plants of the family *Moraceae* (e.g., rubber tree), and plants of the family *Malvaceae* (e.g., cotton).

In a simple method of the present invention, plant tissue (including dormant tissue (including a mature seed, an immature seed, a winter bud, and a tuber), germ plasma, a growing point, and a flower bud) is subjected to electroporation. In the simplest method, a seed is subjected to electroporation. By planting a seed, into which a nucleic acid has been transferred with an electroporation method of the present invention, directly into soil for cultivation, the seed may be readily developed to nucleic acid-transferred matter/transformant. A seed is typically composed of three parts, i.e., an embryo, an endosperm, and a seed coat (Yakichi Noguchi and Shinichiro Kawata, supervisors, Nogaku-Daijiten [Unabridged Dictionary of Agriculture], Yokendo (1987), p. 896 (corresponding portion is prepared by Hideo Chizaka)). An embryo includes all the genetic information of a plant and grows into a whole plant. All monocotyledonous plants and all dicotyledonous plants have embryos. When nucleic acid transfer was performed by the electroporation method of the present invention, it was confirmed that the introduced nucleic acid was expressed in the embryo. Therefore, the simplest method of the present invention can be used to readily obtain a nucleic acid transferred whole plant/transformant in any plants which have a seed including an embryo.

Examples of plants in the *Cruciferae* family include plants in the *Raphanus, Brassica, Arabidopsis, Wasabia,* and *Capsella* genera. Specific examples include Japanese white radish, rapeseed, *Arabidopsis thaliana,* Japanese horseradish, and Shepherd's purse.

Examples of plants in the *Gramineae* family include plants in the *Oryza, Triticum, Hordeum, Secale, Saccharum, Sorghum,* and Zea genera. Specific examples include rice, barley, rye, sugar cane, sorghum, and maize.

As used herein, the term "animal" collectively refers to organisms of the kingdom *Animalia,* which require oxygen and organic food and differ from plants and minerals in the capacity for locomotion. Animals are divided into vertebrates and invertebrates. As vertebrates, Myxiniformes, Petromyzontiformes, Chondrichthyes, Osteichthyes, amphibian, reptilian, avian, mammalian, and the like, may be used. More preferably, mammalian (e.g., Monotremata, Marsupialia, Edentate, Dermoptera, Chiroptera, Carnivore, Insectivore, Proboscidea, Perissodactyla, Artiodactyla, Tubulidentata, Pholidota, Sirenia, Cetacean, Primates, Rodentia, Lagomorpha, etc.) may be used. Evenmore preferably, Primates (e.g., chimpanzee, Japanese macaque, human) are used. Most preferably, cells or organs derived from a human are used. As invertebrates, Crustacea, Diplopoda, Pauropoda, Chilopoda, Symphyla, Insecta, and the like, are used. More preferably, insects (e.g., Lepidoptera, including a silk worm *(Bombyx mori* Linnaeus) and the like) are used.

As used herein, the term "transgenic organism" refers to an organism into which a particular gene is incorporated; the term "transgenic plant" refers to a plant into which a particular gene is incorporated; and the term "transgenic animal" refers to an animal into which a particular gene is incorporated.

Cells and tissue which have been subjected to nucleic acid transfer/transformation by the method of the present invention may be differentiated, grown, and/or multiplied by any known method in the art. In the case of plant species, the step of differentiating, growing, and/or multiplying cells or tissue may be achieved by, for example, cultivating the plant cells or plant tissue or a whole plant including the cells or tissue. Plants can be herein cultivated by any known method in the art. Methods of cultivating plants are illustrated in, for example, "Moderu shokubutsu no Jikken Purotokoru, Ine · Shiroinunazuna : Saibo kogaku Bessatsu Shokubutsu Saibo Kogaku Sirizu 4; Ine no Saibaiho [Experimental Protocol for Model Plants For Rice and Arabidopsis thaliana: Cellular Engineering, Special Issue, Plant Cellular Engineering Series 4; Rice Cultivating Methods]" (Kazutoshi Okuno) pp. 28-32, and "Shiroinunazuna no saibaiho [Cultivating Methods for Arabidopsis]" (Yasuo Niwa) pp. 33-40 (Supervised by Ko Shimamoto and Kiyotaka Okada) , and those skilled in the art can readily carry out. Therefore, these respective methods are not herein described in detail. For example, *Arabidopsis thaliana* can be cultivated by soil culture, rockwoolculture, orhydroponics. When *Arabidopsis thaliana* seeds are disseminated and cultivated under continuous illumination (cool white fluorescent tube (about 6000 lux), the first flower is born about 4 weeks after dissemination and a seed becomes mature about 16 days after flowering. One seedpot contains about 40 to 50 seeds. About 10,000 seeds are obtained before the plant dies about 2 to 3 months after dissemination. In the cultivation of wheat, for example, it is well known that unless wheat seeds are exposed to low temperature and short-day treatment after dissemination, the plant does not ear and flower. Therefore, for example, when wheat is cultivated under an artificial environment (e.g., a green house or a growth chamber), it is necessary to subject the wheat plant at an early developmental stage to low temperature and short-day treatment (e.g., light period: 20°C, 8 hours (about 2000 lux), and dark period: 8°C, 16 hours, etc.). This treatment is called vernalization. Conditions for cultivation required for each plant are generally known in the art , and therefore, need not be herein described in detail.

In the case of species other than plants (e.g., animal species), cells and tissue which have been subjected to nucleic acid transfer/transformation by the method of the present invention may be differentiated, grown, and/or multiplied by any known method in the art (see, for example, Yoshiharu Izumi et al., eds., "Seibutsu Kagaku Jikkenno Tebiki 4. Dobutsu · Soshiki Jikkenho [Guidelines for Biochemical Experiments 4. Experimental Protocol for Animal. Tissue], Kagaku Dojin, 1987, etc.). For example, cells and/or tissue into which a nucleic acid has been transferred by electroporation may be grown with commercially available feed at room temperature (about 25°C).

As used herein, a gene to be transferred is formed of polynucleotides.

As used herein, the terms "polynucleotide", "oligonucleotide" and "nucleic acid" have the same meaning, referring to a polymer of nucleotides of any length. These terms also include "derivative oligonucleotide" or "derivative polynucleotide". The terms "derivative oligonucleotide" and "derivative polynucleotide" are interchangeably used to refer to oligonucleotides or polynucleotides containing a derivative of a nucleotide or having a different link between nucleotides from a normal link. Specifically, examples of such oligonucleotides include 2'-O-methyl-ribonucleotide, derivative oligonucleotides in which a phosphodiester linkage is converted to a phosphorothioate linkage, derivative oligonucleotides in which a phosphodiester linkage is converted to a N3'-P5' phosphoramidate linkage, derivative oligonucleotides in which a ribose and a phosphodiester linkage are converted to a peptide nucleic acid linkage, derivative oligonucleotides in which uracil is substituted with C-5 propynyl uracil, derivative oligonucleotides in which uracil is substituted with C-5 thiazole uracil, derivative oligonucleotides in which cytosine is substituted with C-5 propynyl cytosine, derivative oligonucleotides in which cytosine is substituted with phenoxazine-modified cytosine, derivative oligonucleotides in which ribose is substituted with 2'-O-propylribose, and derivative oligonucleotides in which ribose is substituted with 2'-methoxyethoxyribose. Unless otherwise indicated, a particular nucleic acid sequence also implicitly encompasses conservatively modified variants thereof (e.g., degenerate codon substitutions) and complementary sequences thereof, as well as the sequence explicitly indicated. Specifically, degenerate codon substitutions can be achieved by generating sequences in which the third position of one or more selected (or all) codons is substituted with mixed-base and/or deoxyinosine residues (Batzer et al., Nucleic Acid Res., 19:5081, 1991; Ohtsuka et al. , J. Biol. Chem., 260:2605-2608, 1985; Rossolini et al., Mol. Cell. Probes, 8:91-98, 1994) . The term "nucleic acid" is herein used interchangeably with "gene", "cDNA", "mRNA", "oligonucleotide", and "polynucleotide". A particular nucleic acid sequence also implicitly encompasses "splice variants". Similarly, a particular protein encoded by a nucleic acid implicitly encompasses any protein encoded by a splice variant of that nucleic acid. "Splice variants", as the name suggests, are products of alternative splicing of a gene. After transcription, an initial nucleic acid transcript may be spliced such that different (alternate) nucleic acid splice products encode different polypeptides. Mechanisms for the production of splice variants vary, but include alternate splicing of exons. Alternate polypeptides derived from the same nucleic acid by read-through transcription are also encompassed by this definition. Any products of a splicing reaction, includingrecombinant forms of the splice products, are included in this definition.

As used herein, "gene" refers to a factor defining a hereditary trait. Genes are usually arranged in a predetermined order on a chromosome. A gene defining the primary structure of a protein is called a structural gene. A gene for controlling expression of a structural gene is called a regulatory gene. As used herein, "gene" also refers to "polynucleotide", "oligonucleotide", and "nucleic acid". As used herein, "homology" of a gene refers to the magnitude of identity between two or more gene sequences. Therefore, the greater the homology between two genes, the greater the identity or similarity between their sequences. Whether or not two genes have homology is determined by comparing their sequences directly or by a hybridization method under stringent conditions in the case of a nucleic acid. When two gene sequences are directly compared with each other, the genes have homology if the DNA sequences of the genes have representatively at least 50%, preferably at least 70%, more preferably at least 80%, 90%, 95%, 96%, 97%, 98%, or 99% identity to each other.

A comparison of identity between base sequences and a calculation of homology between base sequences are herein calculated using a sequence analyzing tool BLAST with default parameters.

As used herein, "expression" of gene, polynucleotide, polypeptide, or the like, indicates that the gene or the like is subjected to a certain action *in vivo* and converted into another form. Preferably, a gene, a polynucleotide, or the like is subjected to transcription and translation into a polypeptide form, however, production of mRNA by transcription may be an embodiment of expression. More preferably, the form of such a polypeptide may be obtained by posttranslational processing.

As used herein, "nucleotide" may be naturally occurring or non-naturally occurring. "Derivative nucleotide" or "nucleotide analog" refers to a nucleotide which is different from a naturally-occurring nucleotide but has a function similar to that of the naturally-occurring nucleotide. Such a derivative nucleotide and nucleotide analog are well known in the art. Examples of such a derivative nucleotide and nucleotide analog include, but are not limited to, phosphorothioate, phosphoramidate, methyl-phosphonate, chiral methyl-phosphonate, 2-O-methyl-ribonucleotide, and peptide nucleic acid (PNA).

As used herein, the term "fragment" refers to a polypeptide or polynucleotide having a sequence length of 1 to n-1 with respect to a full-length polypeptide or polynucleotide (its length is n). The length of a fragment may be appropriately changed depending on the purpose. For example, the lower limit of the length of a polypeptide is, for example, 3, 4, 5, 6, 7, 8, 9, 10, 15, 20, 25, 30, 40, 50 and more amino acids. Integers (e.g., 11 and the like), which are not herein explicitly illustrated, may also be appropriate as the lower limit. The lower limit of a polynucleotide is, for example, 5, 6, 7, 8, 9, 10, 15, 20, 25, 30, 40, 50, 75, 100andmorenucleotides. Integers (e.g., 11 and the like), which are not herein explicitly illustrated, may also be appropriate as the lower limit.

General molecular biological techniques used in the present invention can be easily carried out by the those skilled in the art by referencing Ausubel F. A. et al. eds., Current Protocols in Molecular Biology, Wiley, New York, NY, 1988: Sambrook J. et al., Molecular Cloning: A Laboratory Manual, 2nd Ed., Cold Spring Harbor Laboratory Press, Cold Spring Harbor, NY, 1987, or the like.

When a gene is herein discussed, "vector" refers to a vehicle capable of introducing a polynucleotide sequence of interest into a target cell. Such a vector includes a vector which is capable of self-replicating in host cells, such as prokaryotic cells, yeast cells, animal cells, plant cells, insect cells, individual animals and plants, preferably in plant cells, or being incorporated into the chromosome thereof, and having a promoter positioned at a site suitable for the transcription of a polynucleotide of the present invention.

As used herein, the term "expression vector" refers to a nucleic acid sequence comprising a structural gene and a promoter for regulating expression thereof , and in addition, various regulatory elements in a state that allows them to operate within host cells. The regulatory element may include, preferably, terminators, selectable markers such as drug-resistance genes, and enhancers. It is well known to those skilled in the art that the type of an organism (e.g., a plant) expression vector and the type of a regulatory element may vary depending on the host cell. Examples of selectable markers for screening include, but are not limited to, drug-resistance genes, such as the neo gene encoding the enzyme neomycin phosphotransferase conferring resistance to the antibiotic kanamycin (Beck et al., Gene, 19 :327, 1982); the hyg gene encoding the enzyme hygromycin phosphotransferase conferring resistance to the antibiotic hygromycin (Gritz and Davies, Gene, 25:179, 1983); and the bar gene encoding phosphinothricin acetyl transferase conferring resistance to the herbicide phosphinothricin (EP 242236); the spt gene encoding streptomycin phosphotransferase; a streptomycin resistance gene; and a spectinomycin resistance gene (e.g., H. S. Chawla, Introduction to Plant Biotechnology 2nd:363, Science Publishers, Inc., hard cover, 2002); and selectable marker genes, such as the GUS gene encoding β-glucuronidase (Jeffersonetal., Proc. Natl. Acad. Sci. USA, 6:3901, 1986), a luciferase gene (Ow et al., Science, 234: 856, 1986), and a GFP (green fluorescent protein) coding gene (available from, for example, Funakoshi Co., Ltd., Hongo, Bunkyo-ku, Tokyo, Japan).

Examples of an agent used for screening in the present invention include, but are not limited to, kanamycin, hygromycin, geneticin, gentamicin, streptomycin, and spectinomycin.

"Recombinant vector" refers to a vector which can transfer a polynucleotide sequence of interest to a target cell. Examples of such a vector include vectors which are capable of self replicating or capable of being incorporated into a chromosome within host cells (e.g., plant cells and whole plants), and contain a promoter at a site suitable for transcription of a polynucleotide of the present invention.

Examples of "recombinant vectors" for plant cells include Ti plasmid, tobacco mosaic virus vector, and Gemini virus vector.

A "terminator" is a sequence which is located downstream of a protein-encoding region of a gene and which is involved in the termination of transcription when DNA is transcribed into mRNA, and the addition of a poly A sequence. It is known that a terminator contributes to the stability of mRNA, and has an influence on the amount of gene expression. Examples of such a terminator include, but are not limited to, a CaMV35S terminator, a terminator for the nopaline synthase gene (Tnos), and a terminator for the tobacco PR1a gene. As used herein, a "promoter" is a base sequence which determines the initiation site of transcription of a gene and is a DNA region which directly regulates the frequency of transcription. Transcription is started by RNA polymerase binding to a promoter. A promoter region is usually located within about 2 kbp upstream of the first exon of a putative protein coding region. Therefore, it is possible to estimate a promoter region by predicting a protein coding region in a genomic base sequence using a DNA analyzing software. A putative promoter region is usually located upstream of a structural gene. Preferably, a putative promoter region is located within about 2 kbp upstream of the translation initiation site of the first exon.

When mentioning gene expression in the present specification, "site specificity" generally refers to the expression specificity of a gene with respect to a site (e.g., in the case of plants; roots, stems, trunks, leaves, flowers, seeds, germs, embryos, fruits, and the like) within an organism (e.g., plants). "Temporal specificity" refers to the expression specificity of a gene with respect to a developmental stage (e.g., in the case of plants, growth stage, and the number of days after germination) of an organism (e.g., plants). Such specificity can be introduced into a desired organism using an appropriately selected promoter.

As used herein, the term "constitutive" for expression of a promoter of the present invention refers to a character of the promoter that the promoter is expressed in a substantially constant amount in all tissues of an organism no matter whether the growth stage of the organism is a juvenile phase or a mature phase. Specifically, when Northern blotting analysis is performed under the same conditions as those described in examples of the present specification, expression is considered to be constitutive according to the definition of the present invention if substantially the same amount of expression is observed at the same or corresponding site at any time (e.g., two or more time points (e.g., day 5 and day 15)), for example. Constitutive promoters are considered to play a role in maintaining the homeostasis of organisms in a normal growth environment. As used herein, "stress responsive" for promoter expression refers to a character of a promoter that when at least one stress is experienced by an organism, the expression amount of the promoter is changed. Particularly, a character of increasing an expression amount is referred to as "stress inducible". A character of reducing an expression amount is referred to as "stress suppressible". "Stress suppressible" expression is based on the premise that expression is observed in a normal situation. Therefore, this concept overlaps with "constitutive" expression. These characters can be determined by extracting RNA from any portion of an organism and analyzing the expression amount of the RNA by Northern blotting or quantitating expressed proteins by Western blotting. When a plant or a portion thereof (particular cell/tissue, or the like) is transformed with a vector comprising a stress inducible promoter and a nucleic acid encoding a polypeptide of interest, a stimulator having activity of inducing the promoter can be used to cause the particular gene to be expressed under predetermined conditions.

As used herein, the term "enhance" refers to a sequence which is used so as to enhance the expression efficiency of a gene of interest. As such an enhancer for use in plants, an enhancer region containing an upstream sequence within the CaMV35S promoter is preferable. One or more enhancers may be used, or no enhancer may be used.

As used herein, the term "operatively linked" indicates that a desired sequence is located such that expression (operation) thereof is under control of a transcription and translation regulatory sequence (e.g., a promoter, an enhancer, and the like) or a translation regulatory sequence. In order for a promoter to be operatively linked to a gene, typically, the promoter is located immediately upstream of the gene. However, intervening sequences may be present between the promoter and the structural gene, so the promoter is not necessarily adjacent to a structural gene.

The presence of an introduced gene may be confirmed by Southern blotting or PCR. Transcription of an introduced gene may also be detected by Northern blotting or PCR. Expression of a protein, which is a gene product, may be confirmed by, for example, Western blotting, if requried.

Hereinafter, the present invention will be described by way of examples. Examples below are only for purposes of illustration. Therefore, the scope of the present invention is not limited to the above-described explanation or the examples below, except as by the appended claims.

### Examples

### (Methods and Materials)

### (1) Transferred Genes

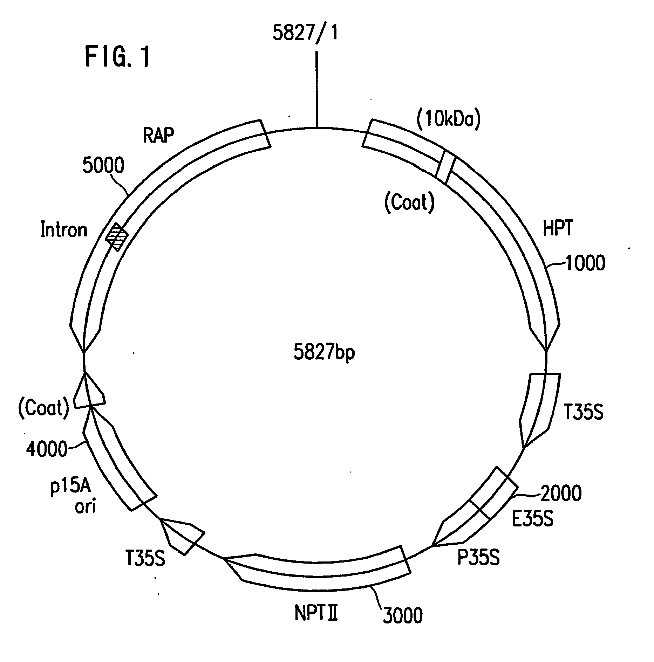

In the present invention, plasmid DNA containing an antibiotics resistant gene and/or the GUS gene was mainly used. The present invention is not limited to this. In examples below, the three plasmids below were used. pBI221 (Clontech) contain the 35S promoter of the cauliflower mosaic virus (CMV), the β glucuronidase (GUS) gene, and the terminator of the nopaline synthase gene(NOS). This plasmid was used mainly for the purpose of monitoring the transfer of a gene based on the GUS activity. pWI-H5K (its restriction map is shown in Figure 1) was constructed by removing the GUS gene from pWI-GUS (Masashi Ugaki et al., Nucleic Acids Res., 19:371-377, 1991) and inserting the NPT II gene thereto. pWI-H5K contains hpt (hygromycin resistant gene), the terminator of CMV, NPT II (kanamycin and gentamicin resistant gene), and the 35S promoter and terminator of CMV. pBC1 has been developed by Dr. Fromm of Cornel University (USA) (at present Dr. Fromm is of Monsanto company). pBC1 contains the promoter and a part of the intron of the alcohol dehydrogenase gene of maize, the GUS gene, the terminator of NOS, the 35S promoter of CMV, and hpt (hygromycin resistant gene), the terminator of NOS (Hagio et al., Plant Cell Rep., 14:329-334, 1995). As plasmid DNA, a plasmid in which the GUS gene in pWI-GUS plasmid is substituted with the GFP (green fluorescent protein) gene, pigA3GFP plasmid (Tamura, Journal of Sericultural Science of Japan, 69:1-12, 2000), or the like, may be used.

### (2) Pretreatment of Seeds (Day 1)

Seed used in the examples were produced by the present inventors or purchased from seed and seedling companies. About 10 to 30 appropriate mature seeds were selected by visual inspection (differing in the size), and were immersed in tap water at 25°C overnight so as to allow the seeds to absorb water. Preferably, tap water used for water absorption contains a sodium hypochlorite aqueous solution for preventing propagation of various bacteria (adjusted to an effective chlorine concentration of about 0.01%). In experiments for the purpose of production of wheat transformants, preferably, water absorption is conducted at 10°C for two nights.

### (3) Electroporation Apparatuses

Electroporation apparatuses used in the present invention may be a commercially available electroporation means (e.g., CUY21EDIT gene transfer apparatus, Nepagene Co., Ltd., Ichikawa-shi, Chiba-ken, Japan).

In this example, an electroporation chamber for use in electroporation may be one having any size such that it can accommodate plant tissue to be transformed. A chamber capable of being cooled is preferable. In this example, a customized electroporation chamber having platinum electrodes and having dimensions of length 1 cm x width 2 cm x height 2 cm was used.

### (4) Preparation of Buffer Solution (Day 2)

In each experiment, 1. 5 ml or less of buffer solution and 150 µg of plasmid DNA were used. The buffer solution was composed of 250 mM calcium chloride, 12.5 mM spermidine, 0.5% polyvinyl pyrrolidone, and 0.3% Tween20.

### (5) Depressurization

The buffer solution and the seeds which had begun sprouting were placed in a petri dish of diameter 35 mm x depth 10 mm, followed by depressurization using a pressure 0.096 MPa or 0.06 MPa below an atmospheric pressure for 1h.

### (6) Electroporation

After depressurization, the seeds and the buffer solution were transferred from the petri dish to the chamber which was in turn allowed to stand on ice for 1 min . Thereafter, a pulse voltage of 100 V or 50 V having a pulse width of 50 µsec (i.e., a rectangular voltage wave where a voltage is applied for 50 µsec and voltage application is rested for 75 µsec and this cycle is repeated) was applied between electrodes where the distance between the electrodes was 1 cm. The number of pulses was 50 or 99. The chamber was further allowed to stand on ice for 2 min. Thereafter, the buffer solution was removed and the seeds were placed back into the original petri dish. 2 ml of 0.5% polyvinyl pyrrolidone (PVP) aqueous solution was poured into the petri dish containing the seeds. The PVP aqueous solution contained a sodium hypochlorite aqueous solution, which was adjusted to an effective chlorine concentration of about 0.01%, for prevention of propagation of various bacteria.

The petri dish was preserved at 4°C for about 1 h and was allowed to stand at 25°C overnight.

### (7) Aftertreatment of Seeds (Day 3)

On the next day, the buffer solution was removed and 2 ml of a new buffer solution having the same composition was added to the petri dish. The petri dish was allowed to stand at 25°C overnight.

### (8) GUS Analysis (Day 4)

On the next day, the buffer solution was removed and 2 ml of X-Gluc solution (100 mM phosphate buffer solution, pH 7.0, 0.05% X-Gluc (5-bromo-4-chloro-3-indolyl-β-D-glucuronide cyclohexyl ammonium salt), 0.5 mM potassium ferricyanide, 0.5 mM potassium ferrocyanide, 0.3% Triton X-100, 20% methanol) was added to the petri dish. The petri dish was allowed to stand at 25°C overnight. Expression of the GUS gene was confirmed on day 5 and thereafter by staining.

### (9) Screening using Antibiotics-containing Medium (Day 4 and thereafter)

The seeds were grown without GUS analysis as follows.
a) the seeds were transferred to a petri dish of 9 cm x 15 mm in which a piece of filter paper was laid down.
b) 10 ml of distilled water solution containing antibiotics was added to the petri dish. When wheat seeds were screened with geneticin, the concentration thereof was 2000 ppm or 1200 ppm. When rice seeds were screened with geneticin, the concentration thereof was 200 ppm.
c) the seeds were grown in a culture room under conditions: (light period: 25°C, 16 h, about 2000 lux; and dark period: 25°C, 8 h) or (light period: 20°C, 8 h, about 2000 lux; and dark period: 8°C, 16 h).

### (10) Growth of Transformants (Day 11 and thereafter)

After screening with the antibiotics-containing medium, the plants were planted in pots (flowerpots) of diameter 8.5 cm x height 5.5 cm containing horticultural soil (New Magic Soil; Sakata Seed Corporation, Tsuzuki-ku, Yokohama-shi, Japan) in an isolated growth chamber under conditions: (light period: 15°C, 8 h, about 50000 lux (sodium lamp); and dark period: 15°C, 16 h) or (light period: 20°C, 8 h, about 2000 lux; and dark period: 8°C, 16 h).

### (11) DNA Extraction

The transfer of a gene was confirmed with PCR as follows. Leaves and stems were collected from a transformed plant, and DNA was extracted from the leaves and stems. DNA extraction may be achieved by any well-known technique. A typical DNA extraction technique is the CTAB technique (Hirofumi Uchimiya, "Shokubutsu Idenshi Sosa Manyuaru Toransugenikku Shokubutsu no Tsukurikata [Manual of Plant Genetic Engineering; How To Produce Transgenic Plants]", Kodansha Scientific, pp. 71-74, hardcover, 1989).

### (12) PCR Analysis

After electroporation, DNAwas extracted from leaves of an individual plant on day 14. A pair of primers (5'-ctgcgtgcaatccatcttg-3' (SEQ ID NO.1), 5'-actcgtcaagaaggcgatagaag-3' (SEQ ID NO. 2)) were used in PCR for detection of the NPT II gene. Another pair of primers (5'-catgattgaacaagatggattgcacgcaggttctc-3' (SEQ ID NO. 3, 5'-cagaagaactcgtcaagaaggcgatagaaggcgat-3' (SEQ ID NO. 4)) may also be used. This primer pair can be more preferably used to detect the NPT II gene in a more specific manner. As polymerase,AmpliTaq Gold(registered trademark)was used in accordance with the manufacturer's instructions (Perkin Elmer Japan Co., Ltd. (Nishi-ku, Yokohama-shi, Japan)). A thermal cycler used for amplification was used under the following settings:
(a) 95°C for 10 min (1 cycle);
(b) 95°C for 1 min, 64°C for 2 min, and 72°C for 2 min (50 cycles);
(c) 72°C for 7 min (1 cycle); and
(d) preservation at 4°C.

### (13) Southern Blotting Analysis

After electroporation, Southern blotting analysis was conducted for DNA derived from an individual plant which had been confirmed by the PCR analysis in (12) to have the introduced gene. Southern blotting is well known in the art and the conditions therefor may be appropriately selected by those skilled in the art if required. In the Southern blotting analysis, a sequence (shown as SEQ ID NO. 5 in the sequence listing) specific to the introduced NPT II gene was used as a probe.

### (14) Genetic Analysis

Plants which were confirmed by Southern blotting analysis to have the introduced gene, were further grown, followed by self-pollination. As a result, anumberofmature seeds were obtained. Among the seeds, about 10 seeds were selected at random, and were cultivated in pots contaning soil. DNA was extracted from leaves of the young plants. The DNA was used as a template to conduct PCR under conditions as described in (12).

### (Example 1)

### (Transformation of Wheat)

Mature seeds of wheat (variety: Norin 61) were subjected to experiments. The seeds were allowed to absorb water at 25°C overnight. 2 ml of an electroporation buffer solution containing 30 seeds which had begun sprouting and plasmid DNA (200 µg/2 ml) of pWI-GUS (for GUS analysis) or pWI-H5K (for growth) were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the pulse width was 50 µsec and the number of pulses was 50. The chamber was further allowed to stand on ice for 2 min. Thereafter, the seeds and the buffer solution were placed back to the original petri dish. The petri dish was preserved at 4°C for about 1 h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The level of expression of the gene was determined using the GUS activity as an index. The result is shown in Table 1 below.

**Table 1**

| Voltage (V/cm) | Level of Expression of gene | Remarks |
|---|---|---|
| 0 | - | |
| 10 | - | |
| 20 | - | |
| 50 | ± | |
| 75 | ± | |
| 100 | + | |
| 150 | - | Seeds were browning or dead. |
| 200 | - | Seeds were browning or dead. |

According to the above-described result, it will be understood that transformation may be performed with the most efficiency when a voltage of 100 V is applied (note: the distance between electrodes was 1 cm).

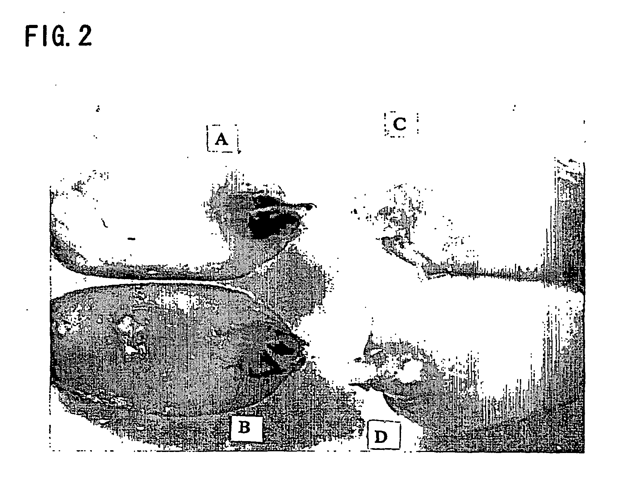

Similar results are shown in Figure 2. Figure 2 shows results of electroporation under conditions: the pulse width was 50 µsec, the number of pulses was 50, the pressure was 0.096 MPa below an atmospheric pressure (depressurization), and the applied voltage was 100 V (A), 50 V (B), 20 V (C), or 0 V (D). When the applied voltage was 100 V, the efficiency of transformation was highest.

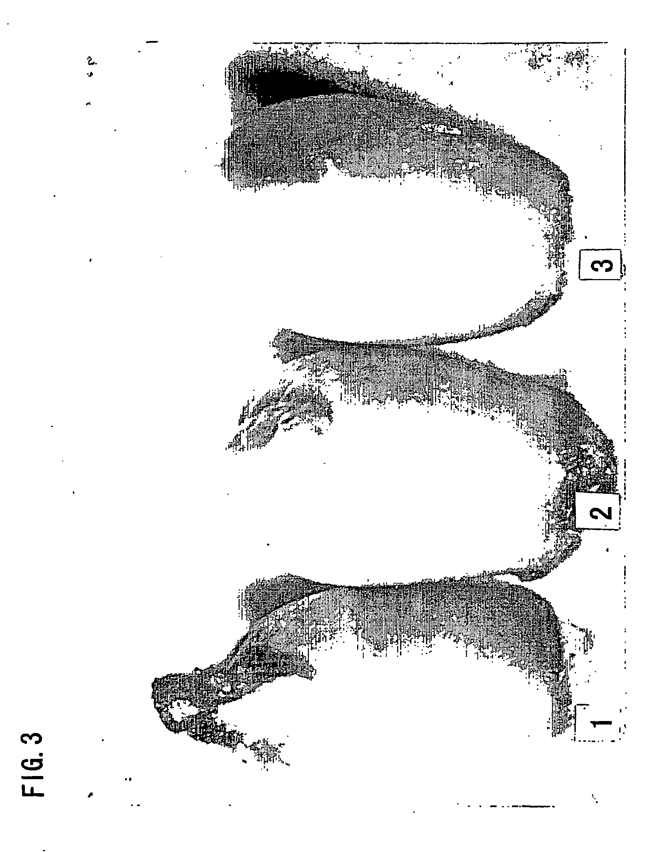

Figure 3 shows a photograph of 1: a wheat seed subjectedtotransformationunderconditions (voltage: 100 V, pulse width: 50 µsec, the number of pulses: 50, pressure: 0.096 MPa below an atmospheric pressure (depressurization); 2: a wheat seed subjected only to electroporation under conditions (voltage: 100 V, pulse width: 50 µsec, the number of pulses 50, no depressurization); and 3: a control wheat seed. As can be seen from Figure 3, transformation was achieved only when depressurization and electroporation were combined.

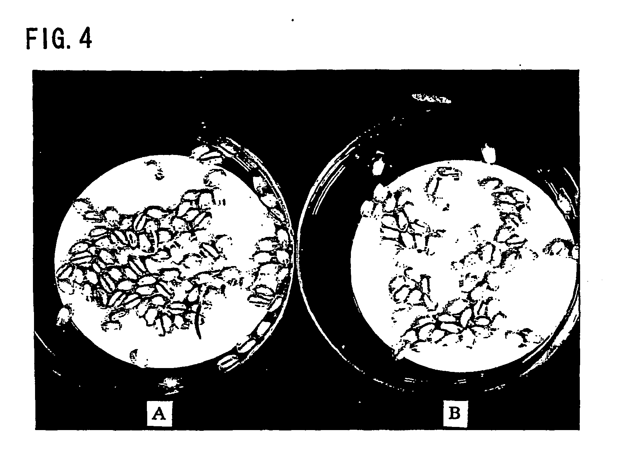

Seeds for growth were not subjected to a GUS activity experiment, and after depressurization, the seeds were grown in distilled water containing 2000 ppm geneticin (as described in (7) aftertreatment of seeds, (9) screening using antibiotics-containing medium, and (10) growth of transformant). The result is shown in Figure 4. DNA was extracted from the seedlings, followed by PCR so as to confirm the presence of the introduced gene (as described in (11) DNA extraction, and (12) PCR Analysis).

SEQ ID NOs. 1 and 2 were used to perform PCR. As a result, it was confirmed that the NPT II gene was introduced into a whole plant.

The reproducibility of the above-described experiments was investigated by repeating the experiments in substantially the same manner. As a result, substantially the same results were obtained. The details of the specific procedure of this repetition experiment are described below. Initially, a piece of filter paper having a diameter of 7 cm was placed in a petri dish of 90 x 15 mm. 150 seeds of wheat Norin 61 were placed on the filter paper. 10 ml of water was added to the petri dish, which was in turn placed in the dark place at 10°C for 2 days, allowing the seeds to absorb water. The added water contained a sodium hypochlorite solution for prevention of propagation of various bacteria (adjusted to an effective chlorine concentration of about 0.01%). On day 2, a young root and a young shoot broke out of the seed coat. Thus, it was confirmed that the seeds had begun sprouting. On this day 2, the seeds were well washed with tap water. Immediately after washing, the nucleic acid transfer treatment of the present invention was performed. 2 ml of an electroporation buffer solution containing 30 wheat seeds and pWI-H5K DNA (200 µg/2 ml) were placed in a petri dish. Depressurization was performed at 0.096 MPa below an atmospheric pressure for 1 h. Thereafter, the seeds and the buffer solution were transferred from the petri dish to a chamber, which was in turn allowed to stand on ice for 1 min. Thereafter, an electric pulse was applied under the following conditions: the voltage was 100 V, where the distance between electrodes was 1 cm; the pulse width was 50 µsec; and the number of pulses was 50. The chamber was further placed on ice for 2 min. Thereafter, the seeds and the buffer solution were placed back in the original petri dish. The petri dish was preserved at 4°C for about 1 h, and was allowed to stand at 25°C overnight. On the next day, the buffer solution was removed, and 2 ml of distilled water was added to the petri dish, which was in turn was allowed to stand at 25°C overnight. This process was performed in a total of 5 times, so that a total of 150 wheat seeds were subjected to the nucleic acid transfer treatment of the present invention.

Next, 10 ml of 0.5% polyvinyl pyrrolidone (PVP) aqueous solution was placed as a browning prevention agent in the 90 x 15-mm petri dish. The wheat seeds subjected to the nucleic acid transfer treatment were placed in the petri dish. The PVP aqueous solution contained a sodium hypochlorite solution for prevention of propagation of various bacteria (adjusted to an effective chlorine concentration of about 0.01%). The petri dish was placed in the dark place at 10°C for 2 days. After two days, 10 ml of aqueous solution containing 1200 ppm geneticin was placed in a new 90 x 15 mm petri dish and the above-described wheat seeds were transferred to this petri dish. The geneticin aqueous solution contained a sodium hypochlorite solution for prevention of propagation of various bacteria (adjusted to an effective chlorine concentration of about 0.02%). Next, the seeds in the petri dish was allowed to grow for 2 weeks under conditions for vernalization in a culture room (light period: 20°C, 8 h, about 2000 lux; and dark period: 8°C, 16 h).

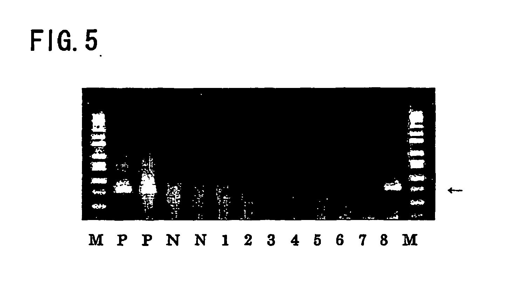

Further, surviving wheat plants which sprouted in the above-described screening process using geneticin antibiotics, were transplanted into a pot containing soil, and were allowed to grow for about 3 to 4 months. This growth was conducted in the same culture room as described above under conditions (light period: 20°C, 8 h, about 2000 lux; and dark period: 8°C, 16 h). About 1 month after potting, leaves were collected from the grown wheat plants. DNA was extracted from the collected leaves. The extracted DNA was used as a template to perform PCR. As a result, the presence of the introduced gene was confirmed. This PCR analysis was conducted using the primer pair and the amplification conditions described in (12) PCR analysis. The results are shown in Figure 5.

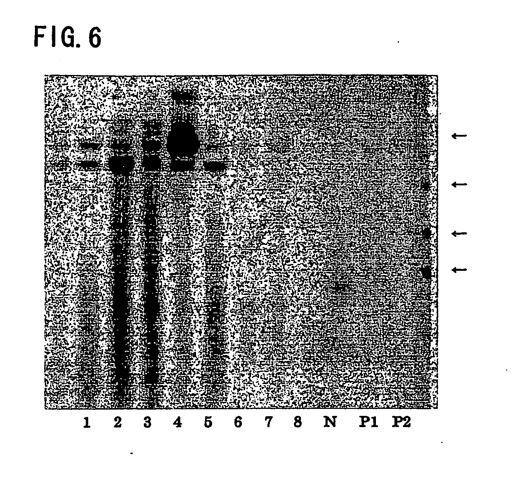

About 5,000 wheat seeds were subjected to similar treatment. As a result, 205 individual plants having geneticin resistance were obtained. Among them, 87 individuals showed the presence of the introduced gene in PCR analysis. These 87 individuals showing the presence of the introduced gene in PCR were grown, and thereafter, were subjected to Southern blotting analysis. 8 individuals were randomly selected from the 87 individuals. DNA was extracted from the 8 individuals and was subjected to Southern blotting analysis. As a result, the presence of the introduced NPT II gene was confirmed in all of the 8 individuals. The results are shown in Figure 6.

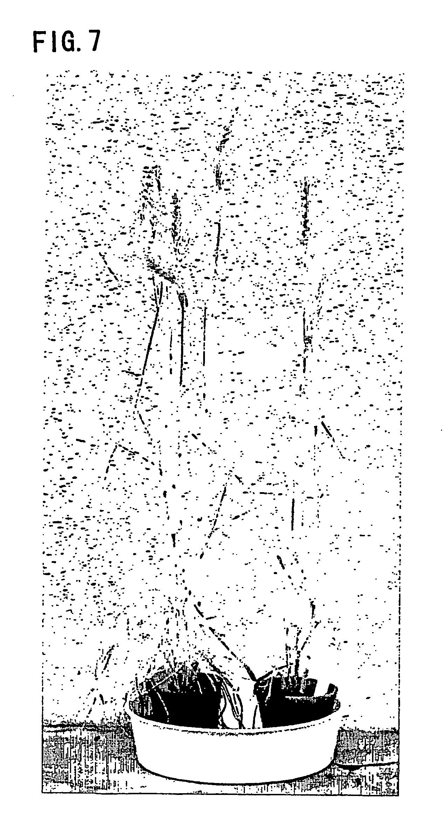

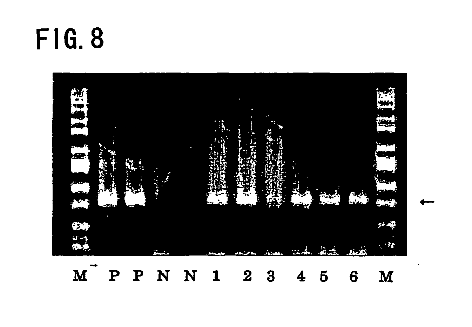

These 8 individuals showing the presence of the introduced gene in Southern blotting analysis were further grown. As a result, it was found that all of the 8 individuals having seed fertility. A photograph of these wheat transformants having seed fertility is shown in Figure 7. Seeds (second generation: T1 generation) obtained from these wheat transformants (first generation: T0 generation) were disseminated in soil. The seeds were grown to young plants. DNA was extracted from leaves of 6 young plants and was subjected to PCR analysis. As a result, the presence of the introduced gene was confirmed for the 6 young plants. This PCR analysis was conducted using the primer pair and amplification conditions described in (12) PCR analysis. The results are shown in Figure 8. As can be seen from Figure 8, it was confirmed that all of the 6 young plants showed the presence of the introduced NPT II gene. Thus, it was demonstrated that the nucleic acid transfer method of the present invention can permit an exogenous gene of interest to be incorporated into a genome in a stable manner and the introduced exogenous gene can be transferred to next generations.

### (Example 2)

Mature seeds of Japonicarice (variety: Koshihikari) were used as material.

The seeds were allowed to absorb water at 25°C overnight. 2 ml of an electroporation buffer solution containing 30 seeds which had begun sprouting and plasmid DNA (200 µg/2 ml) of pWI-GUS or pWI-H5K were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 50 V where the distance between electrodes was 1 cm, the pulse width was 50 µsec, and the number of pulses was 99. The chamber was further allowed to stand on ice for 2 min. Thereafter, the seeds and the buffer solution were placed back to the original petri dish. The petri dish was preserved at 4°C for about 1h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The level of expression of the gene was determined using the GUS activity as an index. The result is shown in Table 2 below.

**Table 2**

| Voltage (V/cm) | Level of Expression of gene | Remarks |
|---|---|---|
| 0 | - | |
| 10 | - | |
| 20 | ± | |
| 50 | + | |
| 75 | ± | Seeds were browning or poorly growing. |
| 100 | - | Seeds were browning or dead. |
| 150 | - | Seeds were browning or dead. |
| 200 | - | Seeds were browning or dead. |

According to the above-described result, it will be understood that transformation may be performed with the most efficiency when a voltage of 50 V is applied (note: the distance between electrodes was 1 cm).

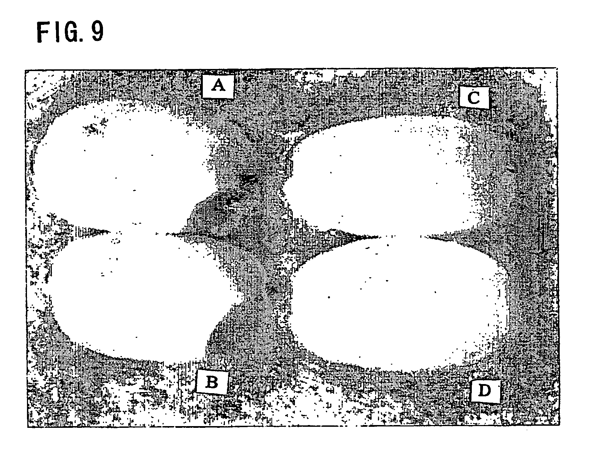

Similar results are shown in Figure 9. Figure 9 shows results of electroporation under conditions: the pulse width was 50 µsec, the number of pulses was 99, the pressure was 0.096 MPa below an atmospheric pressure (depressurization), and the applied voltage was 50 V(A), 20 V (B), 10 V (C), or 0 V (D). When the applied voltage was 50 V, the efficiency of transformation was highest.

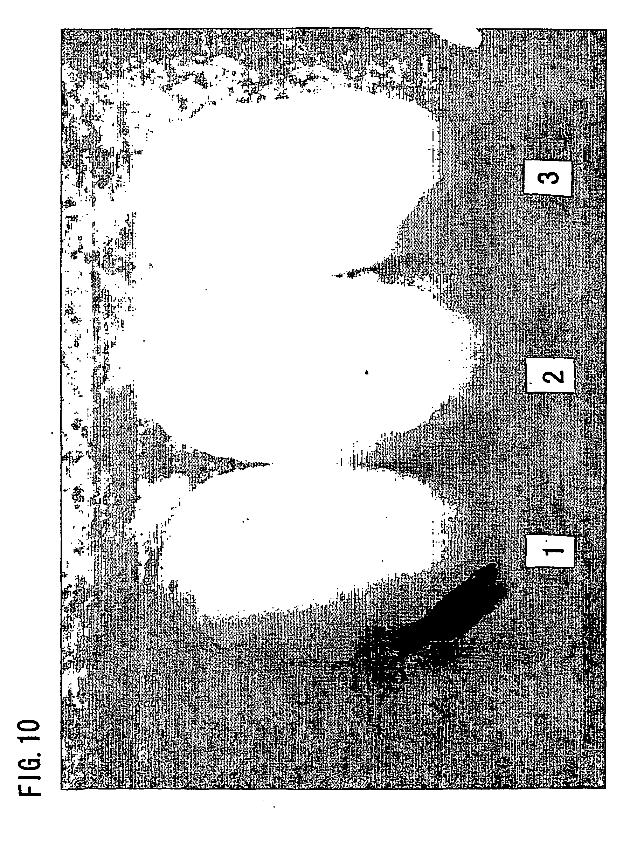

Figure 10 shows a photograph of 1: a rice seed subjected to transformation under conditions (voltage: 50 V, pulse width: 50 µsec, the number of pulses: 99, pressure: 0.096 MPa below an atmospheric pressure(depressurization); 2: a rice seed subjected only to electroporation under conditions (voltage: 50 V, pulse width: 50 µsec, the number of pulses 99, no depressurization); and 3: a control rice seed. As can be seen from Figure 10, transformation was achieved only when depressurization and electroporation were combined.

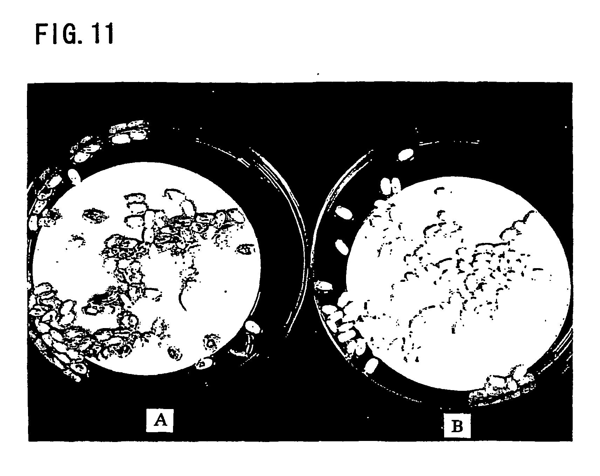

Seeds for growth were not subjected to a GUS activity experiment, and after depressurization, the seeds were grown in distilled water containing 200 ppm geneticin (as described in (7) aftertreatment of seeds, (9) screening using antibiotics-containing medium, and (10) growth of transformant). The result is shown in Figure 11.

The reproducibility of the above-described experiments was investigated by repeating the experiments in substantially the same manner. As a result, substantially the same results were obtained. The details of the specific procedure of this repetition experiment are described below. Initially, a piece of filter paper having a diameter of 7 cm was placed in a petri dish of 90 x 15 mm. 100 seeds of rice (brown rice) were placed on the filter paper. 10 ml of water was added to the petri dish, which was in turn placed at 25°C in a light period of 16 h for 1 day, allowing the seeds to absorb water. The added water contained a sodium hypochlorite solution for prevention of propagation of various bacteria (adjusted to an effective chlorine concentration of about 0.01%). On the next day, the seeds were well washed with tap water. Immediately after washing, the nucleic acid transfer treatment of the present invention was performed. 1 ml of an electroporation buffer solution containing 20 rice seeds and pWI-H5K DNA (100 µg/2 ml) were placed in a petri dish. Depressurization was performed at 0.096 MPa below an atmospheric pressure for 1h. Thereafter, the seeds and the buffer solution were transferred from the petri dish to a chamber, which was in turn allowed to stand on ice for 1 min. Thereafter, an electric pulse was applied by electroporation under the following conditions: the voltage was 50 V where the distance between electrodes was 1 cm; the pulse width was 50 µsec; and the number of pulses was 99. The chamber was further placed on ice for 2 min. Thereafter, the seeds and the buffer solution was placed back in the original petri dish. The petri dish was preserved at 4°C for about 1h, and was allowed to stand at 25°C overnight. On the next day, the buffer solution was removed, and 2 ml of distilled water was added to the petri dish, which was in turn was allowed to stand at 25°C overnight. This process was performed in a total of 5 times, so that a total of 100 rice seeds were subjected to the nucleic acid transfer treatment of the present invention.

Next, 10 ml of 0.5% polyvinyl pyrrolidone (PVP) aqueous solution was placed as a browning prevention agent in the 90 x 15-mm petri dish. The rice seeds subjected to the nucleic acid transfer treatment were placed in the petri dish. The PVP aqueous solution contained a sodium hypochlorite solution for prevention of propagation of various bacteria (adjusted to an effective chlorine concentration of about 0.01%). The petri dish was placed at 25°C in a light period of 16 h for 2 days. After two days, 10 ml of aqueous solution containing 200 ppm geneticin was placed in a new 90 x 15-mm petri dish and the above-described rice seeds wer transferred to this petri dish. The geneticin aqueous solution contained a sodium hypochlorite solution for prevention of propagation of various bacteria (adjusted to an effective chlorine concentration of about 0.02%). Next , the seeds in the petri dish were allowed to grow at 25°C in a light period of 16 h for 2 weeks.

Further, surviving rice plants which sprouted in the above-described screening process using geneticin antibiotics, were transplanted into a pot containing soil, and were allowed to grow for about 3 to 4 months. This growth was conducted at 25°C in a closed green house under typical cultivation conditions. About 1 monthafterpotting, leaves were collected from the grown rice plants. DNA was extracted from the collected leaves. The extracted DNA was used as a template to perform PCR. As a result, the presence of the introduced gene was confirmed. This PCR analysis was conducted using the primer pair and the amplification conditions described in (12) PCR analysis. The PCR analysis confirmed the presence of the NPT II gene.

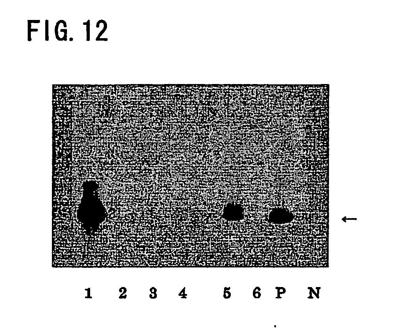

About 2,000 rice seeds were subjected to similar treatment. As a result, 55 individual plants having geneticin resistance were obtained. Among them, 33 individuals showed the presence of the introduced gene in PCR analysis. These 33 individuals showing the presence of the introduced gene in PCR were grown, and thereafter, were subjected to Southern blotting analysis. 6 individuals were randomly selected from the 33 individuals. DNA was extracted from the 6 individuals and was subjected to Southern blotting analysis. As a result, the presence of the introduced NPT II gene was confirmed in 2 individuals. The results are shown in Figure 12.

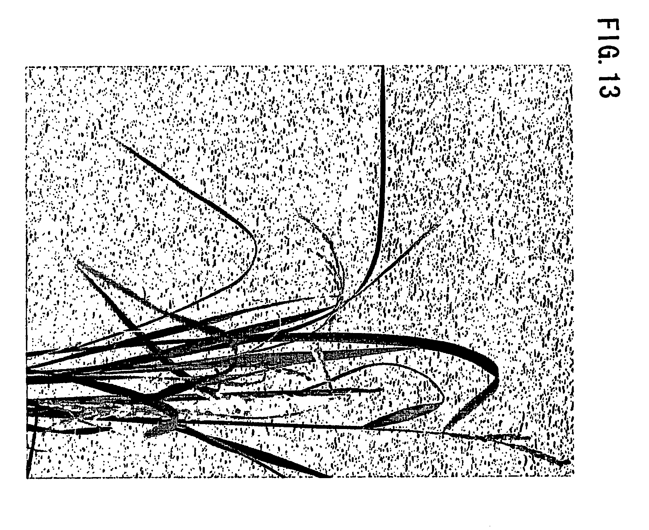

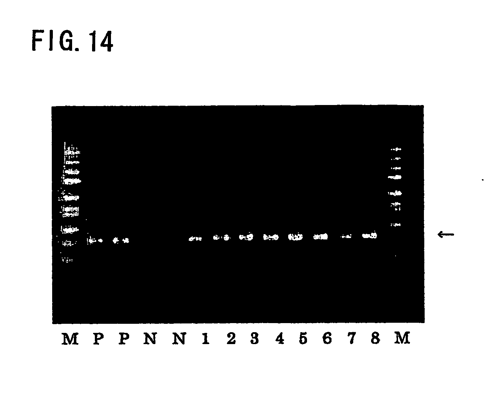

The rice individuals showing the presence of the introduced gene in Southern blotting analysis were further grown. As a result, it was found that the rice individuals hadseedfertility. A photograph of these rice transformants having seed fertility is shown in Figure 13. Seeds (second generation: T1 generation) obtained from these rice transformants (first generation: T0 generation) were disseminated in soil. The seeds were grown to young plants. DNA was extracted from leaves of 8 young plants and was subjected to PCR analysis. As a result, the presence of the introduced gene was determined. This PCR analysis was conducted using the primer pair and amplification conditions described in (12) PCR analysis. The results are shown in Figure 14. As can be seen from Figure 14, it was confirmed that all of the 8 young plants showed the presence of the introduced NPT II gene. Thus, it was demonstrated that the nucleic acid transfer method of the present invention can permit an exogenous gene of interest to be incorporated into a genome in a stable manner and the introduced exogenous gene can be transferred to the next generation.

### (Example 3)

Mature seeds of *Indica* rice (variety: IR24) were used as material.

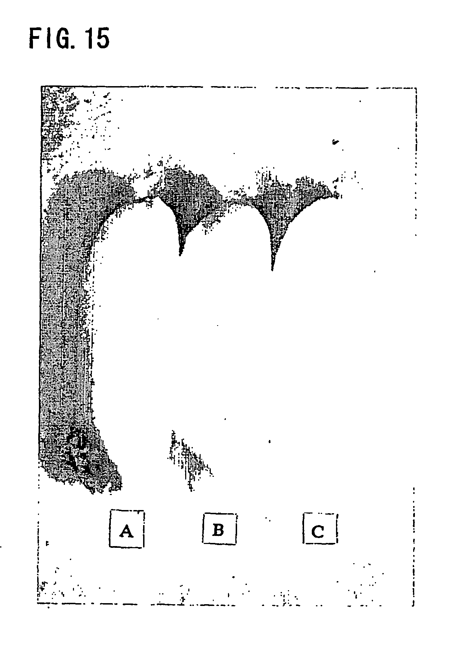

The seeds were allowed to absorb water at 25°C overnight. 2 ml of an electroporation buffer solution containing 30 seeds which had begun sprouting and plasmid DNA (200 µg/2 ml) of pWI-GUS were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 50 V where the distance between electrodes was 1 cm, the pulse width was 50 µsec, and the number of pulses was 99. The chamber was further allowed to stand on ice for 2 min. Thereafter, the seeds and the buffer solution were placed back to the original petri dish. The petri dish was preservedat 4°C for about 1h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results are shown in Figure 15. As can been from Figure 15, transformation was achieved only when depressurization and electroporation were combined.

After depressurization, the seeds were screened using distilled water containing 200 ppm geneticin.

### (Example 4)

Mature seeds of soybean (variety: Oosuzu) were used as material. The seeds were allowed to absorb water at 25°C overnight.

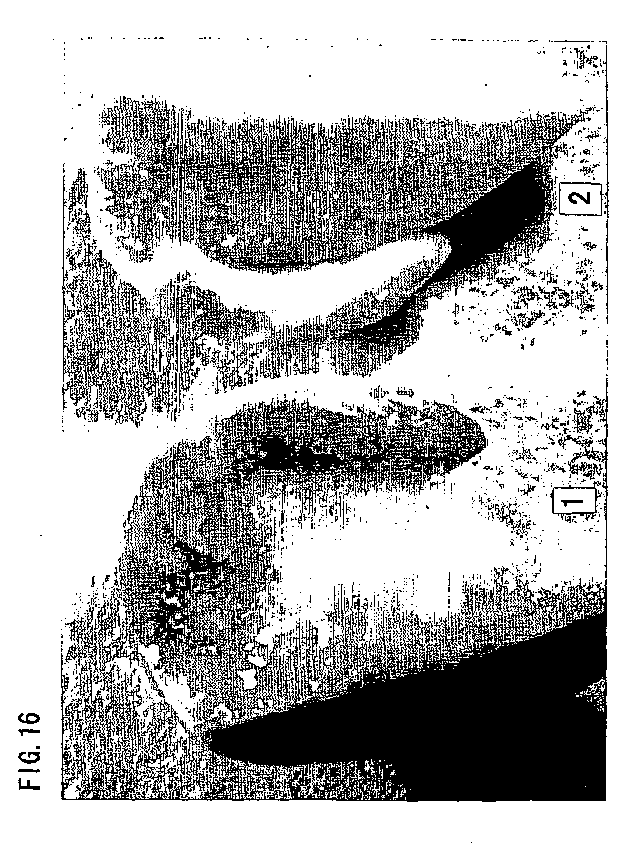

2 ml of an electroporation buffer solution containing 10 seeds which had begun sprouting and plasmid DNA (100 µg/2 ml) of pBCl or pWI-GUS were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 100 V where the distance between electrodes was 1 cm, the pulse width was 50 µsec, and the number of pulses was 50. The chamber was further allowed to stand on ice for 2 min. Thereafter, the seeds and the buffer solution were placed back to the original petri dish. The petri dishwas preserved at 4°C for about 1 h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results in which transformation was successful are shown in Figure 16.

### (Example 5)

Mature seeds of maize (variety: Petercorn) were used as material. The seeds were allowed to absorb water at 25°C overnight. 2 ml of an electroporation buffer solution containing 3 seeds which had begun sprouting and plasmid DNA (100 µg/2 ml) were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 100 V where the distance between electrodes was 1 cm, the pulse width was 50 µsec, and the number of pulses was 50. The chamber was further allowed to stand on ice for 2 min. Thereafter, the seeds and the buffer solution were placed back to the original petri dish. The petri dish was preserved at 4°C for about 1h, and thereaf ter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight.

### (Example 6)

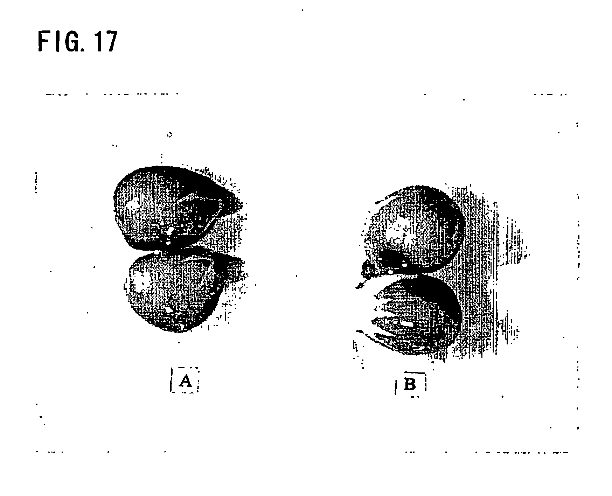

Mature seeds of Chinese cabbage (variety: Muso) were used as material. The seeds were allowed to absorb water at 25°C overnight. 2 ml of an electroporation buffer solution containing 30 seeds which had begun sprouting and plasmid DNA (100 µg/2 ml) were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 100 V where the distance between electrodes was 1 cm, the pulse width was 50 µsec, and the number of pulses was 50. The chamber was further allowed to stand on ice for 2 min. Thereafter, the seeds and the buffer solution were placed back to the original petri dish. The petri dish was preserved at 4°C for about 1h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results in which transformation was successful are shown in Figure 17.

Seeds for growth are not subjected to a GUS activity experiment, and after depressurization, the seeds are grown in distilled water containing an appropriate amount of geneticin (as described in (7) aftertreatment of seeds, (9) screening using antibiotics-containing medium, and (10) growth of transformant). DNA is extracted from the seedlings, followed by PCR so as to confirm the presence of the introduced gene (as described in (11) DNA extraction, and (12) PCR Analysis). Individuals which show the presence of the introduced gene in PCR are further grown, and thereafter, are subjected to Southern blotting. DNA is extracted from some of the grown individuals which are randomly selected, followed by Southern blotting analysis.

### (Example 7)

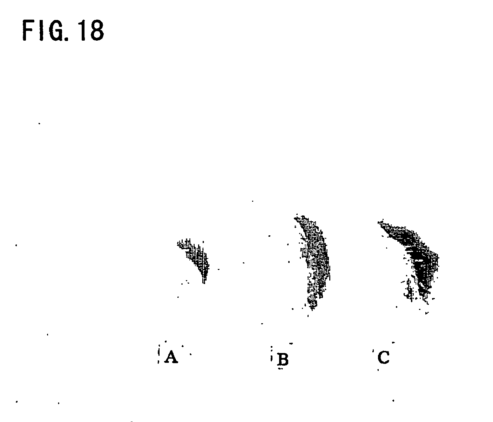

Mature seeds of tomato (variety: Minicarol) were used as material. The seeds were allowed to absorb water at 25°C overnight. 2 ml of an electroporation buffer solution containing 30 seeds which had begun sprouting and plasmid DNA (100 µg/2 ml) were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 100 V where the distance between electrodes was 1 cm, the pulse width was 50 µsec, and the number of pulses was 50. The chamber was further allowed to stand on ice for 2 min. Thereafter, the seeds and the buffer solution were placed back to the original petri dish. The petri dish was preserved at 4°C for about 1 h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results in which transformation was successful are shown in Figure 18.

### (Example 8)

Mature seeds of Japanese cantaloupe (variety: Kinsho melon) are used as material. The seeds are allowed to absorb water at 25°C overnight. The seeds begin sprouting.

2 ml of an electroporation buffer solution containing 40 seeds and plasmid DNA (100 µg/2 ml) are placed in a petri dish. Depressurization is performed at 0.096 MPa below an atmospheric pressure for 1 h. Thereafter, the seeds and the buffer solution are transferred from the petri dish to a chamber. The chamber is placed on ice for 1 min. Thereafter, an electric pulse is applied under the following conditions: the voltage is 100 V where the distance between electrodes is 1 cm, the pulse width is 50 µsec, and the number of pulses is 50.

The chamber is further allowed to stand on ice for 2 min. Thereafter, the seeds and the buffer solution are placed back to the original petri dish.

The petri dish is preserved at 4°C for about 1 h, and thereafter, is allowed to stand at 25°C overnight. On the next day, the buffer solution is removed. 2 ml of distilled water is added to the petri dish, which is in turn allowed to stand at 25°C overnight. On the further next day, the distilled water is removed. 2 ml of X-Gluc solution is added to the petri dish, which is in turn allowed to stand at 25°C overnight.

### (Example 9)

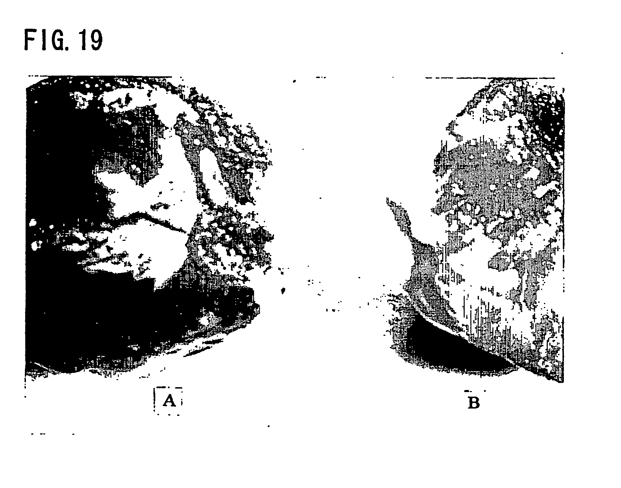

Mature seeds of morning glory (variety: "Sun Smile" and "Youzirotairin") were used as material. The seeds were allowed to absorb water at 25°C overnight. 2 ml of an electroporation buffer solution containing 10 seeds which had begun sprouting and plasmid DNA (100 µg/2 ml) were placed in a petri dish. Depressurization was performed at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 100 V where the distance between electrodes was 1 cm, the pulse width was 50 µsec, and the number of pulses was 50. The chamber was further allowed to stand on ice for 2 min. Thereafter, the seeds and the buffer solution were placed back to the original petri dish. The petri dishwas preserved at 4°C for about 1h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results in which transformation was successful are shown in Figure 19.

### (Example 10)

Mature seeds of *Arabidopsis thaliana* (variety: Col-0) are used as material. The seeds are allowed to absorb water at 25°C overnight. 2 ml of an electroporation buffer solution containing 1000 seeds which have begun sprouting and plasmid DNA (100 µg/2 ml) are placed in a petri dish. Depressurization is performed at 0.096 MPa below an atmospheric pressure for 1 h. Thereafter, the seeds and the buffer solution are transferred from the petri dish to a microtube-type chamber. The chamber is placed on ice for 1 min. Thereafter, an electric pulse is applied under the following conditions: the voltage is 100 V where the distance between electrodes is 1 cm, the pulse width is 50 µsec, and the number of pulses is 50. The chamber is further allowed to stand on ice for 2 min. Thereafter, the seeds and the buffer solution are placed back to the original petri dish. The petri dish is preservedat 4°C for about 1 h, and thereafter, is allowed to stand at 25°C overnight. On the next day, the buffer solution is removed. 2 ml of distilled water is added to the petri dish, which is in turn allowed to stand at 25°C overnight. On the further next day, the distilled water is removed. 2 ml of X-Gluc solution is added to the petri dish, which is in turn allowed to stand at 25°C overnight.

Seeds for growth are not subjected to a GUS activity experiment, and after depressurization, the seeds are grown in distilled water containing an appropriate amount of geneticin (as described in (7) after treatment of seeds, (9) screening using antibiotics-containing medium, and (10) growth of transformant). DNA is extracted from the seedlings, followed by PCR so as to confirm the presence of the introduced gene (as described in (11) DNA extraction, and (12) PCR Analysis). Individuals which show the presence of the introduced gene in PCR are further grown, and thereafter, are subjected to Southern blotting. DNA is extracted from some of the grown individuals which are randomly selected, followed by Southern blotting analysis.

The *Arabidopsis thaliana* individuals showing the presence of the introduced gene in Southern blotting analysis are further grown to produce *Arabidopsis thaliana* individuals had seed fertility. Seeds (second generation: T1 generation) obtained from these *Arabidopsis thaliana* transformants (first generation: T0 generation) are disseminated in soil. The seeds are grown to young plants. DNA is extracted from leaves of the young plants and is subjected to PCR analysis. As a result, the presence of the introduced gene is determined. This PCR analysis is conducted using the primer pair and amplification conditions described in (12) PCR analysis.

### (Example 11)

Mature seeds of Japanese cantaloupe (variety: Kinsho melon) were used as material. The seeds were allowed to absorb water at 25°C overnight. 2 ml of an electroporation buffer solution containing 30 seeds which had begun sprouting and plasmid DNA (100 µg/2 ml) were placed in a petri dish. The seeds were subjected to treatment without depressurization, with depressurization at 0.06 MPa below an atmospheric pressure for 1 h, and with depressurization at 0.096 MPa below an atmospheric pressure for 1 h. Thereafter, the seeds and the buffer solution were transferred from the petri dish to a chamber. The chamber was placed on ice for 1 min. Thereafter, an electric pulse was applied under the following conditions: the voltage was 100 V where the distance between electrodes was 1 cm, the pulse width was 50 µsec, and the number of pulses was 50. The chamber was further allowed to stand on ice for 2 min. Thereafter, the seeds and the buffer solution were placed back to the original petri dish. The petri dish was preserved at 4°C for about 1 h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results are shown in Table 3.

**Table 3**

| Depresssurization (MPa) | Level of Expression of Gene |
|---|---|
| 0 | -¹ |
| 0.06 | -¹ |
| 0.096 | +² |

| | |
|---|---|
| ¹-: For none of the seeds subjected to nucleic acid transfer, X-Gluc stain was confirmed. ²+ : For 15 ormore seeds among the 30 seeds subjected to nucleic acid transfer, X-Gluc stain was confirmed. | |

In Table 3, the level of expression of the gene was evaluated based on the degree of the staining with X-Gluc solution. As can be seen from the results of Table 3, about 0.096-MPa depressurization was preferable for Japanese cantaloupe.

Next, a voltage preferable for the transformation of Japanese cantaloupe was investigated. Japanese cantaloupe was subjected to nucleic acid transfer under the same conditions as described above (0.096-MPa depressurization), except for the following voltages conditions. The results are shown in Table 4 below.

**Table 4**

| Voltage (V/cm) | Level of Expression of Gene |
|---|---|
| 0 | -¹ |
| 10 | -¹ |
| 20 | -¹ |
| 50 | ±² |
| 75 | ±² |
| 100 | +³ |
| 150 | ±² |
| 200 | ±² |

| | |
|---|---|
| ¹-: For none of the seeds subjected to nucleic acid transfer, X-Gluc stain was confirmed. ²±: For 1 to 14 seeds among the 30 seeds subjected to nucleic acid transfer, a weak level of X-Gluc stain was confirmed. ³+: For 15 or more seeds among the 30 seeds subjected to nucleic acid transfer, X-Gluc stain was confirmed. | |

According to the above-described results, it was demonstrated that transformation can be most efficiently performed when a voltage of about 100 V is applied where the distance between electrodes is about 1 cm.

### (Example 12)

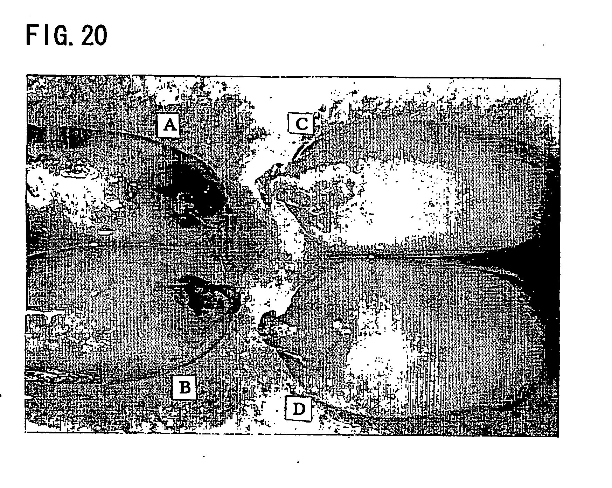

Mature seeds of wheat (variety: Norin 61) were subjected to experiments where conditions for depressurization were compared. The seeds were allowed to absorb water at 25°C overnight. 2 ml of an electroporation buffer solution containing 30 seeds which had begun sprouting and plasmid DNA (100 µg/2 ml) were placed in a petri dish. Sprouting seeds which were placed in 2 ml of an electroporation buffer solution not containing plasmid DNA were used as controls. The seeds were subjected to treatment without depressurization, with depressurization at 0.06 MPa below an atmospheric pressure for 1 h, or with depressurization at 0.096 MPa below an atmospheric pressure for 1 h (note: the control seeds were not subjected to depressurization). Thereafter, the seeds and the buffer solution were transferred from the petri dish to a chamber. The chamber was placed on ice for 1 min. Thereafter, an electric pulse was applied under the following conditions: the voltage was 100 V where the distance between electrodes was 1 cm; the pulse width was 50 µsec; and the number of pulses was 50. The chamber was further allowed to stand on ice for 2 min. Thereafter, the seeds and the buffer solution were placed back to the original petri dish. The petri dish was preserved at 4°C for about 1 h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results are shown in Figure 20. The level of expression of the gene was determined using the degree of staining with X-Gluc solution as an index. As can be seen from Figure 20, in the case of wheat, depressurization at about 0.096 MPa below an atmospheric pressure was preferable. Also, in the case of depressurization at about 0.06 MPa below an atmospheric pressure, X-Gluc stain was confirmed for the wheat seeds.

### (Example 13)

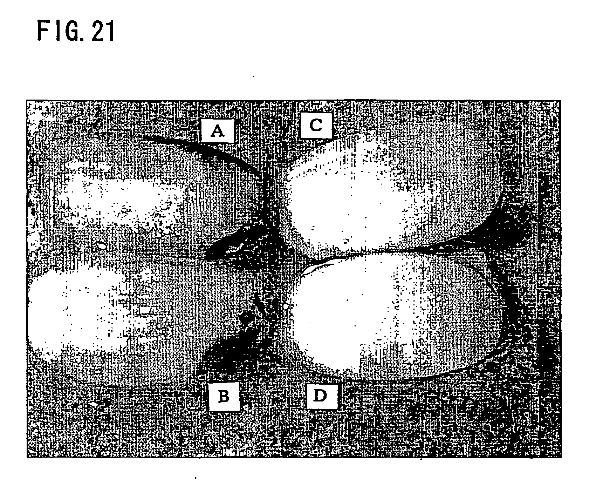

Mature seeds of Japonicarice (variety: Koshihikari) were subjected to experiments where conditions for depressurization were compared. The seeds were allowed to absorb water at 25°C overnight. 2 ml of an electroporation buffer solution containing 30 seeds which had begun sprouting and plasmid DNA (100 µg/2 ml) were placed in a petri dish. Sprouting seeds which were placed in 2 ml of an electroporation buffer solution not containing plasmid DNA were used as controls. The seeds were subjected to treatment without depressurization, with depressurization at 0.06 MPa below an atmospheric pressure for 1 h, or with depressurization at 0.096 MPa below an atmospheric pressure for 1 h (note: the control seeds were not subjected to depressurization). Thereafter, the seeds and the buffer solution were transferred from the petri dish to a chamber. The chamber was placed on ice for 1 min. Thereafter, an electric pulse was applied under the following conditions: the voltage was 50 V where the distance between electrodes was 1 cm; the pulse width was 50 µsec; and the number of pulses was 99. The chamber was further allowed to stand on ice for 2 min. Thereafter, the seeds and the buffer solution were placed back to the original petri dish. The petri dish was preserved at 4°C for about 1 h, and thereafter, was allowed to stand at 25°C overnight. On the next day, the buffer solution was removed. 2 ml of distilled water was added to the petri dish, which was in turn allowed to stand at 25°C overnight. On the further next day, the distilled water was removed. 2 ml of X-Gluc solution was added to the petri dish, which was in turn allowed to stand at 25°C overnight. The results are shown in Figure 21. The level of expression of the gene was determined using the degree of staining with X-Gluc solution as an index. As can be seen from Figure 21, in the case of rice, depressurization at about 0.096 MPa below an atmospheric pressure was preferable. Also, in the case of depressurization at about 0.06 MPa below an atmospheric pressure, X-Gluc stain was confirmed for the rice seeds.

### (Example 14)

### (Transformation of Silk Worm)

Eggs of silk worm (*Bombyx mori* Linnaeus of the phylum *Arthropoda*) are used. 2 ml of an electroporation buffer solution containing 50 silk worm eggs which are arbitrarily selected from dormant eggs preserved in a refrigerator, or 50 silk worm eggs which are arbitrarily selected from non-dormant eggs between 0 and 10 days following oviposition, and plasmid DNA (200 µg/2 ml) are placed in a petri dish. As plasmid DNA, a plasmid, in which the GUS gene in pWI-GUS plasmid is substituted with the GFP (green fluorescent protein) gene, is used. Alternatively, pigA3GFP plasmid (Tamura, Journal of Sericultural Science of Japan, 69:1-12, 2000) is used. The GFP (green fluorescent protein) coding gene (available from, for example, Funakoshi Co., Ltd., Hongo, Bunkyo-ku, Tokyo, Japan) is used. Depressurization is performed at 0.096 MPa below an atmospheric pressure for 5 min. Thereafter, the eggs and the buffer solution are transferred from the petri dish to a chamber. The chamber is placed on ice for 1 min. Thereafter, an electric pulse is applied under the following conditions: the voltage is 50 V where the distance between electrodes is 1 cm, the pulse width is 50 µsec, and the number of pulses is 5. The chamber is further allowed to stand on ice for 2 min.

Thereafter, only the eggs which have been subjected to electroporation are transferred to a petri dish having filter paper. The eggs are allowed to grow at 25°C for 2 to 3 weeks. As feed, commercially available artificial feed (Katakura Kogyo, K. K., Okufu, Sayama-shi, Saitama, Japan) or the like is used. Hatched larvae are irradiated with ultraviolet or blue light to confirm green fluorescence emitted from nucleic acid-transferred matter.

Larvae which have been confirmed to emit green fluorescence are allowed to further grow into adults. The adults are mated with individuals into which the same nucleic acid have been transferred or the like. The next generation individuals are irradiated with ultraviolet or blue light to confirm the inheritance of the introduced nucleic acid by the presence of green fluorescence emitted therefrom.

### (Example 15)

### (Transformation of E. coli)

*E. coli* (prokaryotic organism) is used. 2ml of an electroporation buffer solution containing *E. coli* (about 10⁶to 10⁸ cells/ml) and pBC1 plasmid DNA (10 ng/ml) are placed in a petri dish. Depressurization is performed at 0.096 MPa below an atmospheric pressure for 1 min. Thereafter, the *E. coli* and the buffer solution are transferred from the petri dish to a chamber. The chamber is placed on ice for 1 min. Thereafter, an electric pulse is applied under the following conditions: the voltage is 200 V where the distance between electrodes is 1 cm, the pulse width is 2 µsec, and the number of pulses is 1. The chamber is further allowed to stand on ice for 2 min.

Thereafter, the *E. coli* which has been subjected to electroporation are transferred onto LB agar medium supplemented with 100 ppm ampicillin, followed by incubation at 37°C overnight (see, commercially available experimental guidebooks, such as Molecular Cloning, ver. 2 for LB agar medium). Plasmid DNA and genomic DNA are extracted from the *E. coli*. The presence of the ampicillin resistant gene is confirmed by PCR or the like.

### (Example 16) Electroporation Apparatus and Chamber

Hereinafter, an electroporation apparatus and an electroporation chamber suitable for carrying out the method of the present invention will be described with reference to the accompanying illustrative drawings. The drawings used herein for explanation are only for the purpose of illustration. The scope of the present invention is not intended to be limited by these drawings.

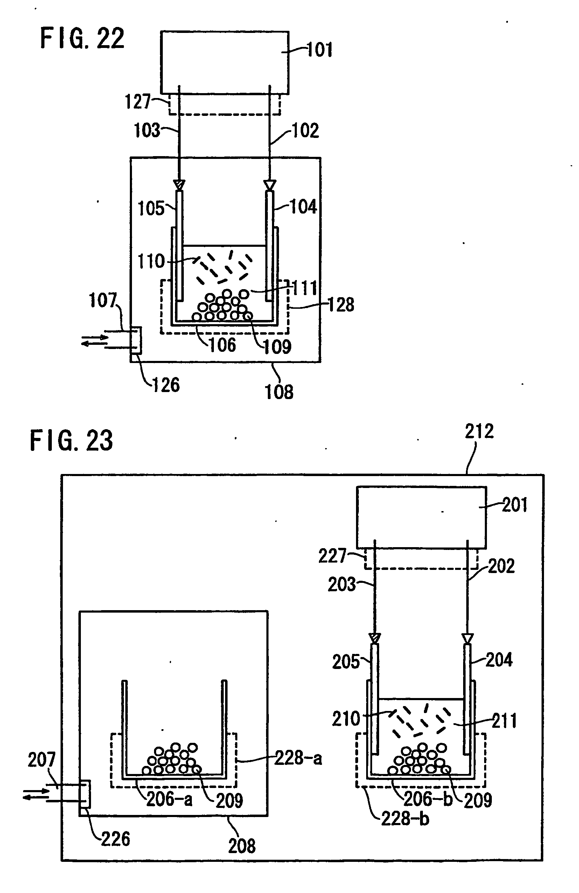

Figure 22 shows an electroporation apparatus according to an embodiment of the present invention. This apparatus comprises a section for holding cells under a pressure different from an atmospheric pressure and an electroporation section. At least a pair of electrodes (104, 105) facing each other are provided in a container (106). Note that although a pair of (two) electrodes (104,105) are depicted in Figure 22, more than a pair of electrodes may be provided in the container (106). Optionally, the container (106) is provided with a temperature control section (128). Cells to be subjected to nucleic acid transfer (109) and nucleic acid molecules to be transferred (110) are placed in the container (106) optionally along with a buffer solution (111). The container (106) may be placed inside another container (108). It is possible to maintain a pressure different from an atmospheric pressure in the container (108). The internal pressure of the container (108) maybe changed by introducing air into (pressurization), or evacuating air from (depressurization), the container (108) through an air hole (107) using a section for maintaining a pressure different from an atmospheric pressure (126). If the container (106) further comprises a lid which is capable of being sealed and an air hole with a section for maintaining a pressure different from an atmospheric pressure, the container (106) and the container (108) may be the same (i. e., the container (106) may substitute for the container (108)). The apparatus further comprises a section for generating a high voltage pulse (101). The high voltage pulse generation section (101) is connected via two cords (102,103) to a pair of electrodes (104,105), respectively. When the high voltage pulse generation section (101) is turned on to generate a high voltage pulse, the electrode (104) and the electrode (105) connected thereto may function as an anode and a cathode, or a cathode and an anode, respectively. Preferably, the high voltage pulse generation section (101) comprises a polarity switching section (127) for reversing the direction of current flow, though it is not necessarily required. By turning the polarity switching section (127) on/off, it is possible to apply a high voltage pulse to the cells (109) and the nucleic acid (110) in two directions.

Figure 23 shows another example of an electroporation apparatus suitable for carrying out the method of the present invention. This apparatus comprises a section for holding cells under a pressure different from an atmospheric pressure and an electroporation section, which are separately installed within a single container (212). In this apparatus, the section for holding cells under a pressure different from an atmospheric pressure comprises a container (206-a). Cells (209) to be subjected to nucleic acid transfer are placed in the container (206-a). A nucleic acid to be transferred and/or a buffer solution are optionally placed in the container (206-a) in addition to the cells (209). The container (206-a) may be placed within another container (208). The inside of the container (208) can be maintained at a pressure different from an atmospheric pressure. The internal pressure of the container (208) may be changed by introducing air into (pressurization), or evacuating air from (depressurization), the container (208) through an air hole (207) using a section for maintaining a pressure different from an atmospheric pressure (226). If the container (206-a) further comprises a lid which is capable of being sealed and an air hole with a section for maintaining a pressure different from an atmospheric pressure, the container (206-a) and the container (208) may be the same (i.e., the container (206-a) may substitute for the container (208)).

The cells which have been subjected to depressurization and/or pressurization by the above-described section, are in turn treated by the electroporation section. The electroporation section comprises a container (206-b). The container (206-b) may be the same or different from the container (206-a). In other words, the container (206-a) used for depressurization and/or pressurization may be used as the container (206-b). Optionally, the containers (206-a, 206-b) are provided with a temperature control section (228-a/228-b). At least one pair of electrodes (204, 205) facing each other are provided inside the container (206-b). Note that although a pair or (two) electrodes (204, 205) are depicted in Figure 23, more than a pair of electrodes may be provided in the container (206-b). The cells (209) to be subjected to nucleic acid transfer and nucleic acid molecules to be transferred (210) are placed in the container (206-b) optionally along with a buffer solution (211). The apparatus further comprises a section for generating a high voltage pulse (201). The high voltage pulse generation section (201) is connected via two cords (202, 203) to a pair of electrodes (204, 205), respectively. When the high voltage pulse generation section (201) is turned on to generate a high voltage pulse, the electrode (204) and the electrode (205) connected thereto may function as an anode and a cathode, or a cathode and an anode, respectively. Preferably, the high voltage pulse generation section (201) comprises a polarity switching section (227) for reversing the direction of current flow, though it is not necessarily required. By turning the polarity switching section (227) on/off, it is possible to apply a high voltage pulse to the cells (209) and the nucleic acid (210) in two directions.

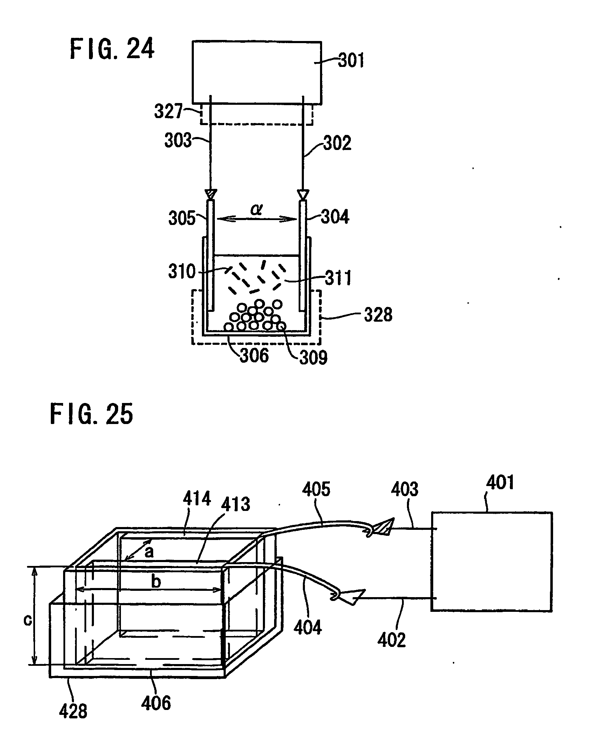

Figure 24 shows another electroporation apparatus suitable for carrying out the method of the present invention. This apparatus comprises only an electroporation section for transferring a nucleic acid into cells. This apparatus is used in combination with holding cells under a pressure different from an atmospheric pressure. This apparatus comprises a container (306). At least a pair of electrodes (304,305) are provided inside the container (306). Note that although a pair of (two) electrodes (304,305) are depicted in Figure 24, more than a pair of electrodes may be provided in the container (306). Optionally, the container (306) is provided with a temperature control section (328). Cells (309) to be subjected to nucleic acid transfer and nucleic acid molecules to be transferred (310) are placed in the container (306) optionally along with a buffer solution (311). The cells (309) have been subjected to depressurization and/or pressurization by any other means. The apparatus further comprises a section for generating a high voltage pulse (301). The high voltage pulse generation section (301) are connected via two cords (302,303) to a pair of electrodes (304,305), respectively. When the high voltage pulse generation section (301) is turned on to generate a high voltage pulse, the electrode (304) and the electrode (305) connected thereto may function as an anode and a cathode, or a cathode and an anode, respectively. Preferably, the high voltage pulse generation section (301) comprises a polarity switching section (327) for reversing the direction of current flow, though not necessarily required. By turning the polarity switching section (327) on/off, it is possible to apply a high voltage pulse to the cells (309) and the nucleic acid (310) in two directions. In the apparatus, the distance (a) between the electrode (304) and the electrode (305) is enough to accommodate a plant seed. This distance is defined as described above.

Figure 25 shows a rectangular parallelepiped electroporation chamber suitable for use in the method of the present invention. This chamber is made of a material capable of resisting a pressure different from an atmospheric pressure (e. g., polypropylene or the like). At least one pair of electrodes (413,414) facing each other are provided inside an electroporation chamber (406) so that the electrodes are closely attached to the inner wall of the chamber. Note that although a pair of (two) electrodes (413,414) are depicted in Figure 25, more than a pair of electrodes may be provided in the container (406). The electrodes (413,414) are preferably in the form of plates disposed along two inner walls facing each other. A line (404) and a line (405), which are made of the same or different metal from the electrodes (413,414), optionally extend from the electrode (413) and the electrode (414). The line (404) and the line (405) are connected to a cord (402) and a cord (403), respectively, which are connected to a high voltage pulse generation section (401). The line (404) and the line (405) may not be provided. If not, the cord (402) and the cord (403) connected to the high voltage pulse generation section (401) maybe connected directly to the electrode (413) and the electrode (414). When the high voltage pulse generation section (401) is turned on to generate a high voltage pulse, the connected electrode (413) and electrode (414) may function as an anode and a cathode or a cathode and an anode, respectively. The chamber has an enough size to accommodate a plant seed. The size of the chamber is represented by the inner dimensions, i. e., length (a) x width (b) of a horizontal cross section thereof x height (c). The size which permits a plant seed to be accommodated is defined as described above. Preferably, the size of the chamber capable of accommodating a plant seed is length 1 cm x width 2 cm x height 2 cm. Cells to be subjected to nucleic acid transfer and nucleic acid molecules to be transferred are placed, optionally along with a buffer solution, in the container (406). Optionally, the container (406) is provided with a temperature control section (428).

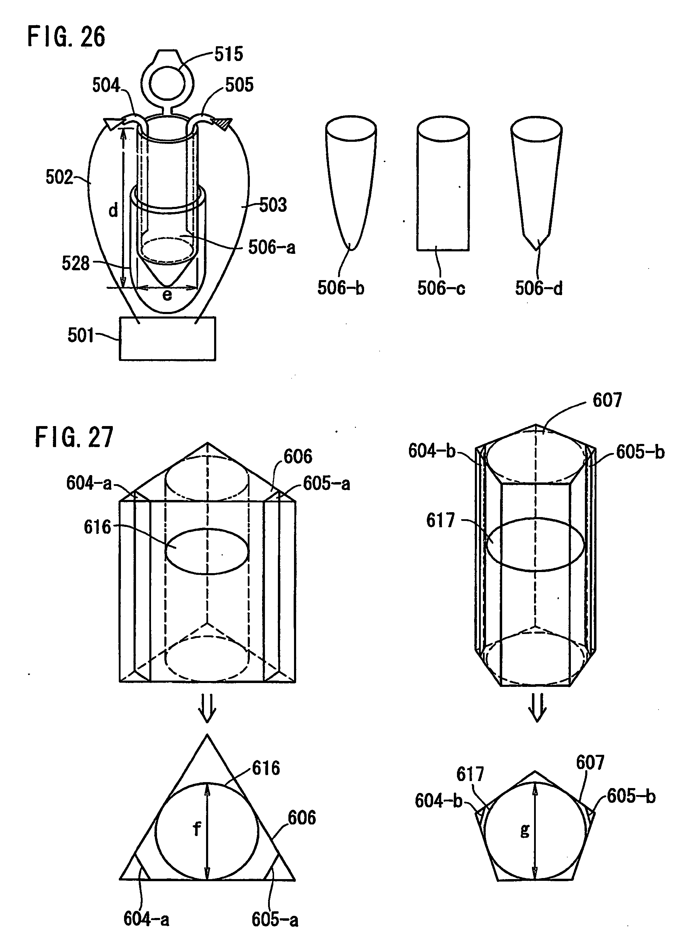

Figure 26 shows a microtube type electroporation chamber suitable for use in the present invention. This chamber (506-a) is made of a material capable of resisting a pressure different from an atmospheric pressure (e. g., polypropylene or the like). At least one pair of electrodes (504,505) facing each other are provided inside a chamber (506-a) so that the electrodes are closely attached to the inner wall of the chamber. Note that although a pair of (two) electrodes (504,505) are depicted in Figure 26, more than a pair of electrodes may be provided in the container (506-a). The electrodes (504,505) are preferably fabricated by attaching stainless foil (about 5 x 40 mm (thickness: about 0.1 mm)) on the inner wall of the microtube with an adhesive. In a certain embodiment, an end of each electrode (504,505) protrudes from the microtube (506-a). The ends of the electrode (504) and the electrode (505) are connected to a cord (502) and a cord (503), respectively, which are connected to a high voltage pulse generation section (501). When the high voltage pulse generation section (501) is turned on to generate a high voltage pulse, the connected electrode (504) and electrode (505) may function as an anode and a cathode or a cathode and an anode, respectively. The chamber has an enough size to accommodate a plant seed. The size of the chamber is represented by the inner dimensions, i. e., diameter (e) of a horizontal cross section thereof x height (d). The size which permits a plant seed to be accommodated is defined as described above. Preferably, the size of the chamber capable of accommodating a plant seed is diameter 1 cm x height 4 cm. Cells to be subjected to nucleic acid transfer and nucleic acid molecules to be transferred are placed, optionally along with a buffer solution, in the container (506-a). The microtube (506-a) optionally comprises a lid (515) for hermetically closing the microtube. Optionally, the container (506-a) is provided with a temperature control section (528).

In the above-described example, the microtube (506-a) has a body having a uniform horizontal section and then tapers to the bottom as shown in Figure 26. The microtube type electroporation chamber may be in any other form. For example, the microtube chamber may have a body having a non-uniform horizontal cross section and a shape tapering toward the bottom (506-b). The chamber may have a body having a uniform horizontal cross section from the top to the bottom (506-c). Alternatively, the chamber may have a body having a non-uniform horizontal cross section as shown in 506-d.

The chamber may be in any shape as long as it has a size which permits it to accommodate a plant seed. Whether or not the chamber has a size which permits it to accommodate a plant seed, may be confirmed by measuring the diameter of an inscribed circle touching the inner wall of the chamber. This inscribed circle touches at least three arbitrary points on the inner wall of the chamber. For example, as shown in Figure 27, when the chamber is in the shape of a triangular prism (606), the diameter (f) of the greatest inscribed circle (616) touching three points as shown in Figure 27 is the size that permits the chamber to accommodate a plant seed. For example, at least a pair of electrodes (604-a, 605-a) are provided in the triangular prism chamber as shown in Figure 27. Note that although a pair of (two) electrodes (604-a, 605-a) are depicted in the triangular prism chamber of Figure 27, more than a pair of electrodes may be provided in the container (606). Similarly, as shown in Figure 27, when the chamber in the shape of a pentangular prism (607), the diameter (g) of the greatest inscribed circle (617) touching five points as shown in Figure 27 is the size that permits the chamber to accommodate a plant seed. For example, at least a pair of electrodes (604-b, 605-b) are provided in the pentangular, prism chamber as shown in Figure 27. Note that although a pair of (two) electrodes (604-b, 605-b) are depicted in the pentangular prism chamber of Figure 27, more than a pair of electrodes may be provided in the container (607). In the case of chambers having other shapes, a size (diameter) of the greatest inscribed circle is the size which permits the chamber to accommodate a plant seed.

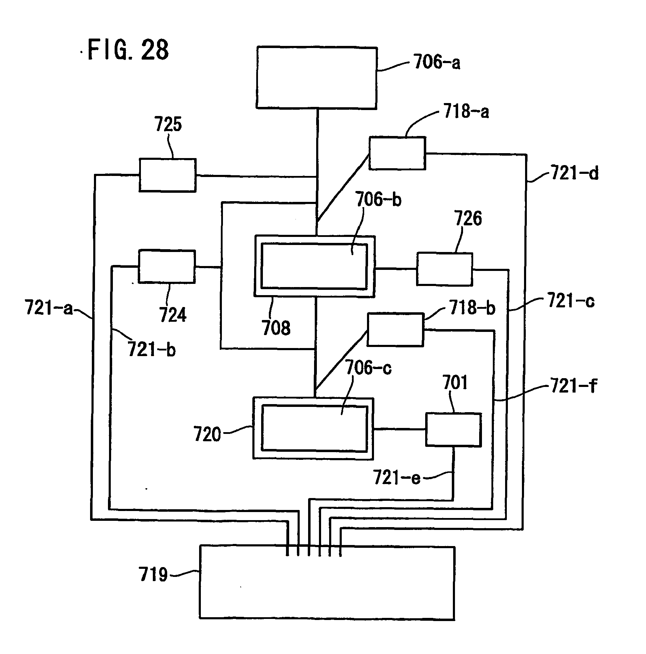

Figure 28 is a schematic diagram showing an automatic electroporation apparatus suitable for carrying out the method according to the present invention. The apparatus comprises :
a) a container (706-a) for placing a mixture containing nucleic acid molecules and cells;
b) a section (724) for placing the nucleic acid molecules in the container of a) ;
c) a section (725) for placing the cells in container of a);
d) a container (708) for holding the cells under a pressure different from an atmospheric pressure, the container being capable of resisting the pressure different from the atmospheric pressure;
e) a section (718-a) for placing the cells in the container of d);
f) a section (726) for maintaining the container of d) at the pressure different from the atmospheric pressure;
g) a container (720) for applying a high voltage pulse to the mixture containing the nucleic acid molecules and the cells;
h) a section (718-b) for placing the mixture containing the nucleic acid molecules and the cells in the container of g) ;
i) a section (701) for applying a high voltage pulse to the mixture containing the nucleic acid molecules and the cells in the container of g); and
j) a section (719) for automatically operating section b), c), e), f), h), and i).

Initially, the container (706-a) is prepared. Cells are injected from the section (725) to the container (706-a). Optionally, nucleic acid molecules may be injected from the section (724) to the same container (706-a) before, substantially simultaneously with, or after injection of the cells. The cells (and, optionally, the nucleic acid molecules) or the container (706-a) containing the injected cells, is placed in the container (708) by action of the section (718-a). The container (708) is made of a material capable of resisting a pressure different from an atmospheric pressure (e.g., polypropylene, etc.). A container (706-b) having the injected cells (and, optionally, nucleic acid molecules) is installed in the container (708). In some cases, the container (708) and the container (706-b) may be the same container. After the cells have been placed in the container (708), the section (726) is activated. With the section (726), the inside of the container (708) is maintained at a pressure different from an atmospheric pressure. Therefore, the cells (and, optionally, nucleic acid molecules) placed in the container (708) are subjected to depressurization and/or pressurization. The cells, which have been subjected to depressurization and/or pressurization, or the container containing the cells, is transferred by a placement section (718-b) from the container (708) to a container (720) (a container containing the cells placed in the container (720) is indicated as 706-c (here, the container (720) and the container (706-c) may be the same container)). When the nucleic acid molecules are not contained in the container (706-b/706-c), the nucleic acid molecules are injected from the section (724) to the container (706-b/706-c) before or after actuation of the placement section (718-b). Optionally, a buffer solution may be injected into a container (706-b/706-c) simultaneously, or sequentially with nucleic acid molecules. When a mixture containing the cells and the nucleic acid molecules has been placed in the container (720), the section (701) is actuated. With the section (701), a high voltage pulse is applied to the mixture containing the cells and the nucleic acid molecules in the container (720). As a result, electroporation occurs between the cells and the nucleic acid molecules. The electroporation apparatus further comprises a control section (719) for automatically controlling the above-described sections (724,725, 718-a, 726,718-b, and 701). The control section (719) is connected to the sections (724, 725,718-a, 726, 718-b, and 701) via cords (721-a, 721-b, 721-c, 721-d, 721-e, and 721-f) as shown in Figure 28. Control signals are transmitted through these cords. In the automatic electroporation apparatus, the section (724) and the section (725) may be the same or different from each other. The section (718-a) and the section (718-b) are the same or different from each other. The container (706-a), the container (706-b), and the container (706-c) may be the same or different from one another. Further, depressurization and/or pressurization in the container (708) and electroporation in the container (720) may be performed in any sequence. Preferably, electroporation in the container (720) may be performed after depressurization and/or pressurization in the container (708).

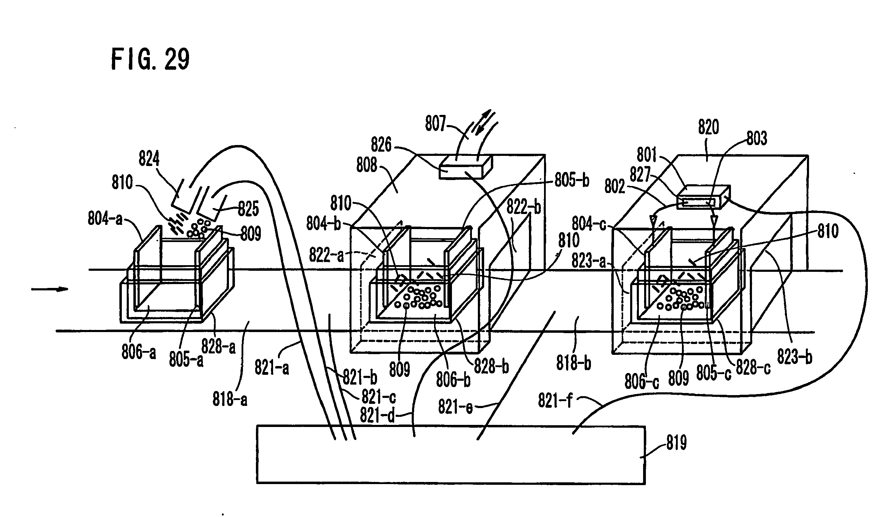

Figure 29 shows a more specific example of automatic electroporation apparatus suitable for carrying out the method of the present invention. In the example of Figure 29, cells are initially placed from a section (825) to a container (806-a). Optionally, at this time of injecting the cells, nucleic acid molecules may be placed from a section (824) to the container (806-a). Optionally, the container (806-a) comprises electrodes (804-a, 805-a). The container (806-a) containing the injected cells (and, optionally, nucleic acid molecules) is transferred by a section (818-a), such as a belt conveyer or the like, into a container (808) through an entrance (822-a) of the container (808). The container containing the injected cells (and, optionally, nucleic acid molecules), which is placed in the container (808), is indicated by 806-b (and, the electrodes provided in the container (806-b) are indicated by 804-b, 805-b). When the cells are placed in the container (808), the entrance (822-a) and an exit (822-b) of the container (808) are closed, so that the container (808) is hermetically closed. In the hermetically closed container (808), a section (826) is actuated so that the inside of the container (808) is maintained at a pressure different from an atmospheric pressure. A pressure different from an atmospheric pressure may be created by by introducing air into (pressurization), or evacuating air from (depressurization), the container (808) through an air hole (807) by action of the section (826). The container (806-b) containing the cells (and, optionally, nucleic acid molecules), which have been subjected to depressurization and/or pressurization as described above, is transferred by a section (818-b), such as a belt conveyer or the like, through the exit (822-b) of the container (808) to a container (820). If at this time the container (806-b/806-c) does not contain nucleic acid molecules, nucleic acid molecules are injected into the container (806-b/806-c) during this transferring step. The container containing the injected cells and nucleic acid molecules, which has been placed in the container (820), is indicated by 806-c (and the electrodes provided in container (806-c) are indicated by 804-c, 805-c). After the cells and the nucleic acid molecules have been placed in the container (820), an entrance (823-a) and exit (823-b) of the container (820) are preferably closed. The container (820) comprises a section (801) for generating a high voltage pulse. The high voltage pulse generation section (801) is connected via two cords (802, 803) to the electrode (804-c) and the electrode (805-c), respectively. When the high voltage pulse generation section (801) is turned on to generate a high voltage pulse, the connected electrode (804-c) and electrode (805-c) may function as an anode and a cathode or a cathode and an anode. Preferably, the high voltage pulse generation section (801) comprises a polarity switching section (827) for reversing current flow, though it is not necessarily required. By switching the polarity switching section (827) on/off, it is possible to apply a high voltage pulse to the cells (809) and the nucleic acid molecules (810) in two directions. Further, the automatic electroporation apparatus comprises a control section (819) for automatically controlling the above-described sections (824,825, 818-a, 826,818-b, 801). The control section (819) is connected to the sections (824, 825,818-a, 826,818-b, 801) via respective cords (821-a, 821-b, 821-c, 821-d, 821-e, 821-f) as shown in Figure 29. Control signals are transmitted through the cords. Note that in Figure 29, the section (824) for placing nucleic acid molecules into the container (806-a) and the section (825) for placing cells into the container (806-a) are depicted as separate sections, though the sections (824,825) may be the same section (i. e., a single section for placing cells and nucleic acid molecules into the container (806-a)). Note that although a pair of (two) electrodes (804-a, 805-a ; 804-b, 805-b ; 804-c, 805-c) are depicted in Figure 29, more than a pair of electrodes may be provided in the container (806-a; 806-b ; 806-c, respectively). Further, in Figure 29, the placement sections (818-a, 818-b) are depicted as a single belt conveyer, though the placement sections (818-a, 818-b) may be separate sections. Further, the container (808) for changing pressure and/or the container (820) for performing electroporation may be moved on the belt conveyer simultaneously with, or instead of, the movement of the containers (806-a, 806-b, 806-c) containing nucleic acid molecules and/or cells. Alternatively, the placement sections may transfer materials/suspensions placed in the container (806-a), the container (806-b), and the container (806-c) by suctioning/drainage using an automatic pump without a belt conveyer or the like. The container (806-a), the container (806-b), and the container (806-c) may be the same or different from one another. When they are different containers, the contents (i.e., cells and/or nucleic acid molecules) are transferred between each container, such as from the container (806-a) to the container (806-b) and/or from the container (806-b) to the container (806-c), or the like. The transfer may be carried out by, for example, simply tilting a container; or suctioning/drainage using an automatic dispenser/automatic pipette/automatic pump or the like. Optionally, the container (806-a, 806-b, 806-c) is provided with a temperature control section (828-a, 828-b, 828-c).

As described above, the present invention has been heretofore illustrated with the preferred embodiments. It should be understood that the scope of the present invention is limited only by the claims.

### INDUSTRIAL APPLICABILITY

A simple and rapid nucleic acid transfer method is provided.

The simple and rapid nucleic acid transfer method of the present invention can be utilized for research and development in the art to easily allow large-scale processing and large-scale analysis. In addition, the present invention triggers dramatic advances in research, potentially leading to development of innovative recombinanants.

## Claims

1. A method for transferring a nucleic acid into a plant cell, comprising the steps of:
a) subjecting the cell to depressurization; and
placing the cell and the nucleic acid under conditions capable of inducing electroporation, which includes applying a high voltage pulse to the cell and nucleic acid,
wherein the depressurization step is performed under a pressure reduced by at least 0.02 MPa from atmospheric pressure.

2. A method according to claim 1, wherein the plant cell is a cell of dormant plant tissue.

3. A method according to claim 2, wherein the dormant plant tissue is a seed.

4. A method for producing a plant, wherein a nucleic acid is transferred into cells of the plant, comprising the steps of:
a) subjecting the cell to depressurisation;
b) placing the cell and the nucleic acid under conditions capable of inducing electroporation, which includes applying a high voltage pulse to the cell and nucleic acid,
wherein the depressurisation step is performed under a pressure reduced by at least 0.02 MPa from atmospheric pressure; and
c) differentiating, growing and/or multiplying the cell.

5. A method according to claim 4, wherein step a) comprises a step of subjecting a seed containing the cell to depressurisation, and step b) comprises a step of placing the seed containing the cell and the nucleic acid under the conditions capable of inducing electroporation.

6. A method according to claim 1 or 4, wherein the plant or plant seed is a monocotyledonous plant or plant seed.

7. A method according to claim 6, wherein the monocotyledonous plant is a plant of the family *Gramineae.*

8. A method according to claim 7, wherein the plant of the family *Gramineae* is wheat (*Triticum aestivam L.*)*.*

9. A method according to claim 7, wherein the plant of the family *Gramineae* is rice (*Oryza sativa L.*)*.*

10. A method according to claim 7, wherein the plant of the family *Gramineae* is maize (*Zea mays L.*)*.*

11. A method according to claim 1 or 4, wherein the plant or plant seed is a dicotyledonous plant or plant seed.

12. A method according to claim 11, wherein the dicotyledonous plant is a plant of the family *Cruciferae.*

13. A method according to claim 12, wherein the plant of the family *Cruciferae* is Chinese cabbage (*Brassica rapa L.*)*.*

14. A method according to claim 12, wherein the plant of the family *Cruciferae* is rape (*Brassica napus L.*)*.*

15. A method according to claim 11, wherein the dicotyledonous plant is a plant of the family *Leguminosae.*

16. A method according to claim 15, wherein the plant of the family *Leguminosae* is soybean (*Glycine max Merr*)*.*

17. A method according to claim 11, wherein the dicotyledonous plant is a plant of the family *Solanaceae*.

18. A method according to claim 17, wherein the plant of the family Solanaceae is tomato (*Lycopersicum esculentum Mill*)*.*

19. A method according to claim 11, wherein the dicotyledonous plant is a plant of the family *Cucurbitaceae.*

20. A method according to claim 19, wherein the plant of the family *Cucurbitaceae* is Japanese cantaloupe (*Cucumis melo L.*)*.*

21. A method according to claim 11, wherein the dicotyledonous plant is a plant of the family *Convolvulaceae.*

22. A method according to claim 21, wherein the plant of the family *Convolvulaceae* is morning glory (*Pharbitis nil Choisy*)*.*

## Patentansprüche

1. Verfahren zum Transferieren einer Nucleinsäure in eine Pflanzenzelle, umfassend die folgenden Schritte:
a) Durchführen einer Druckverminderung mit der Zelle; und
b) Verbringen der Zelle und der Nucleinsäure unter Bedingungen, die Elektroporation zu induzieren vermögen, welche das Anlegen eines Hochspannungsimpulses an die Zelle und Nucleinsäure umfasst,
wobei der Druckverminderungsschritt unter einem Druck durchgeführt wird, der um mindestens 0,02 MPa vom Atmosphärendruck herabgesetzt ist.

2. Verfahren nach Anspruch 1, wobei die Pflanzenzelle eine Zelle von ruhendem Pflanzengewebe ist.

3. Verfahren nach Anspruch 2, wobei das ruhende Pflanzengewebe eine Saat ist.

4. Verfahren zur Produktion einer Pflanze, wobei eine Nucleinsäure in Zellen der Pflanze transferiert wird, umfassend die folgenden Schritte:
a) Durchführen einer Druckverminderung mit der Zelle; und
b) Verbringen der Zelle und der Nucleinsäure unter Bedingungen, die Elektroporation zu induzieren vermögen, welche das Anlegen eines Hochspannungsimpulses an die Zelle und Nucleinsäure umfasst,
wobei der Druckverminderungsschritt unter einem Druck durchgeführt wird, der um mindestens 0,02 MPa vom Atmosphärendruck herabgesetzt ist; und
c) Differenzieren, Wachsenlassen und/oder Vervielfältigen der Zelle.

5. Verfahren nach Anspruch 4, wobei Schritt a) einen Schritt der Durchführung einer Druckverminderung mit einer Saat, die die Zelle enthält, umfasst, und Schritt b) einen Schritt des Verbringens der Saat, die die Zelle und die Nucleinsäure enthält, unter die Bedingungen, die Elektroporation zu induzieren vermögen, umfasst.

6. Verfahren nach Anspruch 1 oder 4, wobei die Pflanze oder Pflanzensaat eine monokotyledone Pflanze oder Pflanzensaat ist.

7. Verfahren nach Anspruch 6, wobei die monokotyledone Pflanze eine Pflanze der Familie *Gramineae* ist.

8. Verfahren nach Anspruch 7, wobei die Pflanze der Familie *Gramineae* Weizen (*Triticum aestivum L.*) ist.

9. Verfahren nach Anspruch 7, wobei die Pflanze der Familie *Gramineae* Reis (*Oryza sativa L.*) ist.

10. Verfahren nach Anspruch 7, wobei die Pflanze der Familie *Gramineae* Mais (*Zea Mays L.*) ist.

11. Verfahren nach Anspruch 1 oder 4, wobei die Pflanze oder Pflanzensaat eine dikotyledone Pflanze oder Pflanzensaat ist.

12. Verfahren nach Anspruch 11, wobei die dikotyledone Pflanze eine Pflanze der Familie *Cruciferae* ist.

13. Verfahren nach Anspruch 12, wobei die Pflanze der Familie *Cruciferae* Chinakohl (*Brassica rapa L.*) ist.

14. Verfahren nach Anspruch 12, wobei die Pflanze der Familie *Cruciferae* Raps (*Brassica napus L.*) ist.

15. Verfahren nach Anspruch 11, wobei die dikotyledone Pflanze eine Pflanze der Familie *Leguminosae* ist.

16. Verfahren nach Anspruch 15, wobei die Pflanze der Familie *Leguminosae* Sojabohne (*Glycine max Merr*) ist.

17. Verfahren nach Anspruch 11, wobei die dikotyledone Pflanze eine Pflanze der Familie *Solanaceae* ist.

18. Verfahren nach Anspruch 17, wobei die Pflanze der Familie *Solanaceae* Tomate (*Lycopersicum esculentum Mill*) ist.

19. Verfahren nach Anspruch 11, wobei die dikotyledone Pflanze eine Pflanze der Familie *Cucurbitaceae* ist.

20. Verfahren nach Anspruch 19, wobei die Pflanze der Familie *Cucurbitaceae* Japanische Cantaloup (Cucumis melo) ist.

21. Verfahren nach Anspruch 11, wobei die dikotyledone Pflanze eine Pflanze der Familie *Convolvulacea* ist.

22. Verfahren nach Anspruch 21, wobei die Pflanze der Familie *Convolvulacea* Prachtwinde (*Pharbitis nil Choisy*) ist.

## Revendications

1. Procédé pour transférer un acide nucléique dans une cellule végétale, comprenant les étapes consistant à :
a) soumettre la cellule à une décompression ; et
b) mettre la cellule et l'acide nucléique dans des conditions pouvant provoquer une électroporation, comprenant l'application d'une impulsion à haute tension à la cellule et l'acide nucléique,
dans lequel l'étape de décompression est effectuée sous une pression réduite d'au moins 0,02 MPa à partir de la pression atmosphérique.

2. Procédé selon la revendication 1, dans lequel la cellule végétale est une cellule de tissu végétal dormant.

3. Procédé selon la revendication 2, dans lequel le tissu végétal dormant est une graine.

4. Procédé pour la production d'une plante, dans lequel un acide nucléique est transféré dans des cellules de la plante, comprenant les étapes consistant à :
a) soumettre la cellule à une décompression ;
b) mettre la cellule et l'acide nucléique dans des conditions pouvant provoquer une électroporation, comprenant l'application d'une impulsion à haute tension à la cellule et l'acide nucléique,
dans lequel l'étape de décompression est effectuée sous une pression réduite d'au moins 0,02 MPa à partir de la pression atmosphérique ; et
c) différencier, cultiver et/ou multiplier la cellule.

5. Procédé selon la revendication 4, dans lequel l'étape a) comprend une étape de soumission d'une graine contenant la cellule à une décompression, et l'étape b) comprend une étape de mise en place de la graine contenant la cellule et l'acide nucléique dans les conditions pouvant provoquer une électroporation.

6. Procédé selon la revendication 1 ou 4, dans lequel la plante ou graine de plante est une plante ou graine de plante monocotylédone.

7. Procédé selon la revendication 6, dans lequel la plante monocotylédone est une plante de la famille des graminées.

8. Procédé selon la revendication 7, dans lequel la plante de la famille des graminées est le blé (*Triticum aestivum L.*)*.*

9. Procédé selon la revendication 7, dans lequel la plante de la famille des graminées est le riz (*Oryza sativa L.*)*.*

10. Procédé selon la revendication 7, dans lequel la plante de la famille des graminées est le maïs (*Zea mays L.*)*.*

11. Procédé selon la revendication 1 ou 4, dans lequel la plante ou graine de plante est une plante ou graine de plante dicotylédone.

12. Procédé selon la revendication 11, dans lequel la plante dicotylédone est une plante de la famille des crucifères.

13. Procédé selon la revendication 12, dans lequel la plante de la famille des crucifères est le chou chinois (*Brassica rapa L.*)*.*

14. Procédé selon la revendication 12, dans lequel la plante de la famille des crucifères est le colza ( *Brassica napus L.*)*.*

15. Procédé selon la revendication 11, dans lequel la plante dicotylédone et une plante de la famille des légumineuses.

16. Procédé selon la revendication 15, dans lequel la plante de la famille des légumineuses est le soja (*Glycine max Merr*)*.*

17. Procédé selon la revendication 11, dans lequel la plante dicotylédone est une plante de la famille des solanacées.

18. Procédé selon la revendication 17, dans lequel la plante de la famille des solanacées est la tomate (*Lycopersicum esculentum Mill*)*.*

19. Procédé selon la revendication 11, dans lequel la plante dicotylédone est une plante de la famille des cucurbitacées.

20. Procédé selon la revendication 19, dans lequel la plante de la famille des cucurbitacées est le cantaloup du Japon (*Cucumis melo L.*)*.*

21. Procédé selon la revendication 11, dans lequel la plante dicotylédone est une plante de la famille des convolvulacées

22. Procédé selon la revendication 21, dans lequel la plante de la famille des convolvulacées est l'ipomée (*Pharbitis nil Choisy*)*.*
